# EUROPEAN PATENT SPECIFICATION

(11) **EP 0 702 084 B1**
(45) Date of publication and mention of the grant of the patent: **22.12.1999**
(21) Application number: 95115441.8
(22) Date of filing: 21.03.1989
(51) Int. Cl.: C12N 15/86, C12N 9/50, C12N 15/11, C12N 9/12, C07K 14/73

(54) **Recombinant retroviruses**
Rekombinante Retroviren
Rétrovirus recombinants

(30) Priority: 21.03.1988 US 170515
(43) Date of publication of application: 20.03.1996
(62) Divisional of application: 89105059.3
(73) Proprietor: CHIRON CORPORATION, Emeryville, California 94608 (US)
(72) Inventor: Gruber, Harry E., San Diego, California 92130 (US); Jolly, Douglas J., Leucadia, California 92024 (US); Respess, James G., San Diego, California 92109 (US); Laikino, Paul K., San Diego, California 92130 (US)
(74) Representative: Goldin, Douglas Michael

(56) References cited:
- EP-A- 0 243 204
- FR-A- 2 559 159
- FR-A- 2 606 030
- SCIENCE, vol. 229, 26 July 1985 LANCASTER, PA US, pages 345-352, J.G.IZANT ET AL. 'Constitutive and conditional suppression of exogenous and endogenous genes by anti-sense RNA'
- NATURE, vol. 318, 5 December 1985 LONDON GB, page 414 R.TELLIER ET AL. 'New strategies for AIDS therapy and prophylaxis'
- PROCEEDINGS OF THE NATIONAL ACADEMY OF SCIENCES OF USA, vol. 83, July 1986 WASHINGTON US, pages 4794-4798, J.T.HOLT ET AL. 'Inducible production of c-fos antisense RNA inhibits 3T3 cell proliferation'

## Description

### Technical Field

The present invention relates generally to retroviruses, and more specifically, to recombinant retroviruses which are capable of delivering vector constructs to susceptible target cells. These vector constructs are typically designed to express desired proteins in target cells, for example, proteins which stimulate immunogenic activity or which are conditionally active in defined cellular environments.

### Background of the Invention

Although bacterial diseases are, in general, easily treatable with antibiotics, very few effective treatments or prophylactic measures exist for many viral, cancerous, and other nonbacterial diseases, including genetic diseases. Traditional attempts to treat these diseases have employed the use of chemical drugs. In general, these drugs have lacked specificity, exhibited high overall toxicity, and thus have been therapeutically ineffective.

Another classic technique for treating a number of nonbacterial diseases involves the elicitation of an immune response to a pathogenic agent, such as a virus, through the administration of a noninfectious form of the agent, such as a killed virus, thereby providing antigens from the pathogenic agent which would act as an immunostimulant.

A more recent approach for treating viral diseases, such as acquired immunodeficiency syndrome (AIDS) and related disorders, involves blocking receptors on cells susceptible to infection by HIV from receiving or forming a complex with viral envelope proteins. For example, Lifson et al. (Science 232:1123-1127, 1986) demonstrated that antibodies to CD4 (T4) receptors inhibited cell fusion (syncytia) between infected and noninfected cD4 presenting cells in vitro. A similar CD4 blocking effect using monoclonal antibodies has been suggested by McDougal et al. (Science 231:382-385, 1986). Alternatively, Pert et al. (Proc. Natl. Acad. Sci. USA 83:9254-9258, 1986) have reported the use of synthetic peptides to bind T4 receptors and block HIV infection of human T-cells, while Lifson et al. (J. Exp. Med. 164:2101, 1986) have reported blocking both syncytia and virus/T4 cell fusion by using a lectin which interacts with a viral envelope glycoprotein, thereby blocking it from being received by CD4 receptors.

A fourth, recently suggested technique for inhibiting a pathogenic agent, such as a virus, which transcribes RNA is to provide antisense RNA which complements at least a portion of the transcribed RNA, and binds thereto, so as to inhibit translation (To et al., Mol. Cell. Biol. 6:758, 1986).

However, a major shortcoming of the techniques described above is that they do not readily lend themselves to control as to the time, location or extent to which the drug, antigen, blocking agent or antisense RNA are utilized. In particular, since the above techniques require exogenous application of the treatment agent (i.e., exogenous to the sample in an in vitro situation), they are not directly responsive to the presence of the pathogenic agent. For example, it may be desirable to have an immunostimulant expressed in increased amounts immediately following infection by the pathogenic agent. In addition, in the case of antisense RNA, large amounts would be required for useful therapy in an animal, which under current techniques would be administered without regard to the location at which it is actually needed, that is, at the cells infected by the pathogenic agent.

As an alternative to exogenous application, techniques have been suggested for producing treatment agents endogenously. More specifically, proteins expressed from viral vectors based on DNA viruses, such as adenovirus, simian virus 40, bovine papilloma, and vaccinia viruses, have been investigated. By way of example, Panicali et al. (Proc. Natl. Acad. Sci. USA 80:5364, 1983) introduced influenza virus hemagglutinin and hepatitis B surface antigens into the vaccinia genome and infected animals with the virus particles produced from such recombinant genes. Following infection, the animals acquired immunity to both the vaccinia virus and the hepatitis B antigen.

However, a number of difficulties have been experienced to date with viral vectors based on DNA viruses. These difficulties include (a) the production of other viral proteins which may lead to pathogenesis or the suppression of the desired protein; (b) the capacity of the vector to uncontrollably replicate in the host, and the pathogenic effect of such uncontrolled replication; (c) the presence of wild-type virus which may lead to viremia; and (d) the transitory nature of expression in these systems. These difficulties have virtually precluded the use of viral vectors based on DNA viruses in the treatment of viral, cancerous, and other nonbacterial diseases, including genetic diseases.

Due to the nontransitory nature of their expression in infected target cells, retroviruses have been suggested as a useful vehicle for the treatment of genetic diseases (for example, see F. Ledley, The Journal of Pediatrics 110:1, 1987). However, in view of a number of problems, the use of retroviruses in the treatment of genetic diseases has not been attempted. Such problems relate to (a) the apparent need to infect a large number of cells in inaccessible tissues (e.g., brain); (b) the need to cause these vectors to express in a very controlled and permanent fashion; (c) the lack of cloned genes; (d) the irreversible damage to tissue and organs due to metabolic abnormalities; and (e) the availability of other partially effective therapies in certain instances.

In addition to genetic diseases, other researchers have contemplated using retroviral vectors to treat nongenetic diseases (see, for example, EP 243,204 - Cetus Corporation; Sanford, J. Theor. Biol.130:469, 1988; Tellier et al., Nature 318:414, 1985; and Bolognesi et al., Cancer Res. 45:4700, 1985).

Tellier et al. suggested protecting T-cell clones by apparently infecting stem cells with "defective" HIV having a genome which could express antisense RNA to HIV RNA. Bolognesi et al. have suggested the concept of generating a nonvirulent HIV strain to infect stem cells so that T4 cells generated therefrom would carry interfering, nonvirulent forms of virus and thereby protect those cells from infection by virulent HIV. However, it would appear that the "attenuated" or "defective" HIV viruses used in both of the foregoing papers could reproduce (i.e., are not replication defective) such that the resulting viruses could infect other cells, with the possibility of an increased risk of recombination with previously present HIV or other sequences, leading to loss of attenuation. Non-nonreplicative forms would necessitate a defective helper or packaging line for HIV. However, since the control of HIV gene expression is complex, such cells have to date not been constructed. Furthermore, as the infecting attenuated or defective virus is not chimeric (a "nonchimeric" retrovirus being one with substantially all of its vector from the same retrovirus species), even if they were made replication defective, for example, by deletion from their genomes of an essential element, there still exists a significant possibility for recombination within the host cells with resultant production of infectious viral particles.

Although Sanford (J. Theor. Biol. 130:469, 1988) has also proposed using a genetic cure for HIV, he notes that due to the potential that exists for creating novel virulent viruses via genetic recombination between natural AIDS virus and therapeutic retroviral vectors carrying anti-HIV genes, retroviral gene therapy for AIDS may not be practical. Similarly, while McCormick & Kriegler (EP 243,204 A2) have proposed using retroviral vectors to deliver genes for proteins, such as tumor necrosis factor (TNF), the techniques they describe suffer from a number of disadvantages.

### Summary of the Invention

Briefly stated, the present invention is directed in part, toward methods for inhibiting a function of a pathogenic agent, such as a virus, through the use of recombinant retroviruses.

More specifically, according to one aspect of the present invention, there is provided a replication-defective recombinant retrovirus for use in a method of therapeutic treatment, said retrovirus carrying a vector construct capable of preventing, inhibiting, stabilizing or reversing an infectious, cancerous or auto-immune disease, said vector construct when in human cells infected with said retrovirus being capable of directing the expression of an enzyme which is not normally expressed in said cells and which converts a compound with little or no cytotoxicity into a cytotoxic compound capable of inhibiting a function of a pathogenic agent necessary for pathogeniciny.

Thus, the present invention provides methods for inhibiting a function of a pathogenic agent necessary for disease, such as diseases caused by viral infections, cancers or immunological abnormalities.

Where a toxic palliative is to be produced by cells containing the recombinant viral genome, either it can be produced from precursors existing within the cells or, additionally, through exogenously providing a precursor to a toxic agent. In either case, the toxic agent will be localized to cells containing the viral construct and another agent associated with the pathogenic condition. For example, the other agent might be a protein produced through transcription and translation of a pathogenic viral genome present in the cell. Specificity for the pathogenic condition can also be attained or further enhanced through targeting of the entry of the recombinant retroviruses to the cells affected by the pathogenic condition. It should be understood in the foregoing discussion, and throughout this application, that when reference is made to the viral construct "expressing" or "producing" any substance in a cell, or the like, this in fact refers to the action of the resulting provirus following reverse transcription of the viral RNA in the cell.

As Indicated above, a recombinant retrovirus of the present invention may be employed in relation to a variety of therapeutic treatments, including methods for diminishing or eliminating an unwanted or deleterious immune response.

Regardless of the means by which the recombinant retrovirus exerts its inhibitory action as described above, the retroviral genome will be "replication defective" (i.e., incapable of reproducing in cells infected with it). Thus, there will be only a single stage of infection in either an in vitro or in vivo application, thereby substantially reducing the possibility of insertional mutagenesis. Preferably, to assist in this end, the recombinant retrovirus lacks at least one of the gag, pol, or env genes. Further, the recombinant viral vector is preferably chimeric (that is, the gene which is to produce the desired result is from a different source than the remainder of the retrovirus). A chimeric construction further reduces the possibility of recombination events within cells infected with the recombinant retrovirus, which could produce a genome that can generate viral particles.

The present invention also provides a method for producing such recombinant retroviruses in which the retroviral genome is packaged in a capsid and envelope, preferably through the use of a packaging cell. The packaging cells are provided with viral protein-coding sequences, preferably in the form of two plasmids, which produce all proteins necessary for production of viable retroviral particles, an RNA viral construct which will carry the desired gene along with a packaging signal which will direct packaging of the RNA into the retroviral particles.

Suitable packaging or packaging cell lines, and the genome necessary for accomplishing such packaging, are described in Miller et al. (Mol. Cell. Bio. 6:2895, 1986). As described by Miller et al., it is preferable that further changes be made to the proviral construct other than simple deletion of the packaging signal in order to reduce the chances of recombination events occurring within the packaging cell line, which may result in production of viral particles which are not replication defective.

By way of example, preparations of vector are made by growing the producer cells in normal medium, washing the cells with PBS plus human serum albumin (HSA) at 10 mg/ml, then growing the cells for 8-16 hours in PBS plus HSA. Titers obtained are typically 10⁴ to 10⁶/ml depending on the vector, packaging line or particular producer line clone. The vector supernatants are filtered to remove cells and are concentrated up to 100-fold by filtration through 100,000 or 300,000 pass Amicon filters (Wolff et al., Proc. Natl. Acad. Sci. 84:3344, 1987). This lets globular proteins of 100,000 or 300,000 pass but retains 99% of the viral vector as infectious particles. The stocks Can be frozen for storage since they lose about 50% of the infectious units on freezing and thawing. The most direct delivery involves administration of the appropriate gene-carrying vector into the individual and reliance upon the ability of the vector to efficiently target to the appropriate cells, The dose would be 10⁵ - 10⁶ infectious units/kg body weight. However, a more practical approach may involve the extracorporeal treatment of patient peripheral blood lymphocytes (PBL), fibroblasts or other cells obtained from each individual with the vector. PBL could be maintained in culture through the use of mitogens (phytohemagglutinin) or lymphokines (e.g., IL-2). This type of approach allows for directed vector infection, monitoring of expression and expansion of the desired enzyme expressing cells prior to injection, and return of vector-expressing cells to the respective patient. Other types of cells could also be explanted, vector introduced, and the cells returned to the patient.

A different form of administration is the implantation of producer lines making retroviral vector particles. These may be immunologically unmatched classical producer cell lines or the patients own cells, which have been explanted, treated and returned (see VI Alternative Viral Vector Packaging Techniques, below). Both types of implants (10⁵ - 10⁶/kg body weight) would have a limited life span in the patient, but would lead to the retroviral vector infecting large numbers (10⁷ - 10¹⁰) of cells in their vicinity in the body.

There are now described below a number of techniques for producing recombinant retroviruses which can facilitiate:
i) the production of higher titres from packaging cells;
ii) packaging of vector constructs by means not involving the use of packaging cells;
iii) the production of recombinant retroviruses which can be targeted for preselected cell lines; and
iv) the integration of the proviral construct into a preselected site or sites in a cell's genome.

One technique for producing higher titres from packaging cells takes advantage of the discovery that of the many factors which can limit titre from a packaging cell, one of the most limiting is the level of expression of the packaging proteins, namely, the gag, pol, and env proteins, as well as the level of expression of the retroviral vector RNA from the proviral vector. This technique allows the selection of packaging cells which have higher levels of expression (i.e., produce higher concentrations) of the foregoing packaging proteins and vector construct RNA. More specifically, this technique allows selection of packaging cells which produce high levels of what is referred to herein as a "primary agent," which is either a packaging protein (e.g., gag, pol, or env proteins) or a gene of interest to be carried into the genome of target cells (typically a vector construct). This is accomplished by providing in packaging cells a genome carrying a gene (the "primary gene") which expresses the primary agent in the packaging cells, along with a selectable gene, preferably downstream from the primary gene. The selectable gene expresses a selectable protein in the packaging cells, preferably one which conveys resistance to an otherwise cytotoxic drug. The cells are then exposed to a selecting agent, preferably the cytotoxic drug, which enables identification of those cells which express the selectable protein at a critical level (i.e., in the case of a cytotoxic drug, by killing those cells which do not produce a level of resistance protein required for survival).

Preferably, in the technique briefly described above, the expression of both the selectable and primary genes is controlled by the same promoter. In this regard, it may be preferable to utilize a retroviral 5' LTR. In order to maximize titre of a recombinant retrovirus from packaging cells, this technique is first used to select packaging cells expressing high levels of all the required packaging proteins, and then is used to select which of these cells, following transfection with the desired proviral construct, produce the highest titres of the recombinant retrovirus.

Techniques are also provided for packaging of vector constructs by means not involving the use of packaging cells. These techniques make use of DNA viruses such as baculovirus, adenovirus, or vaccinia virus, preferably adenovirus. These viruses are known to express relatively high levels of proteins from exogenous genes provided therein. For such DNA virus vectors, recombinant DNA viruses can be produced by in vivo recombination in tissue culture between viral DNA and plasmids carrying retroviral or retroviral vector genes. The resultant DNA viral vectors carrying either sequences coding for retroviral proteins or for retroviral vector RNA are purified into high titre stocks. Alternatively, the constructs can be constructed in vitro and subsequently transfected into cells which provide in trans viral functions missing from the DNA vectors. Regardless of the method of production, high titre (10⁷ to 10¹¹ units/ml) stocks can be prepared that will, upon infection of susceptible cells, cause high level expression of retroviral proteins (such as gag, pol, and env) or RNA retroviral vector genomes, or both. Infection of cells in culture with these stocks, singly or in combination, will lead to high-level production of retroviral vectors, if the stocks carry the viral protein and viral vector genes. This technique, when used with adenovirus or other mammalian vectors, allows the use of primary cells (e.g., from tissue explants or cells such as WI38 used in production of vaccines) to produce recombinant retroviral vectors.

In an alternative to the foregoing technique, recombinant retroviruses are produced by first generating the gag/pol and env proteins from a cell line infected with the appropriate recombinant DNA virus in a manner similar to the preceding techniques, except that the cell line is not infected with a DNA virus carrying the vector construct. Subsequently, the proteins are purified and contacted with the desired viral vector RNA made in vitro, transfer RNA (tRNA), liposomes, and a cell extract to process the env protein into the liposomes, such that recombinant retroviruses carrying the viral vector RNA are produced. Within this technique, it may be necessary to process the env protein into the liposomes prior to contacting them with the remainder of the foregoing mixture. The gag/pol and env proteins may also be made after plasmid mediated transfection in eukaryotic cells, in yeast, or in bacteria.

The technique for producing recombinant retroviruses which can be targeted for preselected cell lines utilizes recombinant retroviruses having an env gene comprised of a cytoplasmic segment of a first retroviral phenotype, and an extracellular binding segment exogenous to the first retroviral phenotype. The binding segment is from a second viral phenotype or from another protein with desired binding properties which is selected to be expressed as a peptide which will bind to the desired target.

Techniques for integrating a retroviral genome at a specific site in the DNA of a target cell involve the use of homologous recombination, or alternatively, the use of a modified integrase enzyme which will recognize a specific site on the target cell genome. Such site-specific insertion allows genes to be inserted at sites on the target cells' DNA, which will minimize the chances of insertional mutagenesis, minimize interference from other sequences on the DNA, and allow insertion of sequences at specific target sites so as to reduce or eliminate the expression of an undesirable gene (such as a viral gene) in the DNA of the target cell.

It will be appreciated that any of the above-described techniques may be used independently of the others in particular situations, or can be used in conjunction with one or more of the remainder of the techniques.

These and other aspects of the present invention will become evident upon reference to the following further description and attached drawings.

### Brief Description of the Drawings

Figure 1 illustrates the construction of the plasmids carrying the vectors TK1 (without SV-Neo) and TK3 (plus SV-Neo).

Figure 2 illustrates the construction of the plasmid carrying the vector KTVIHAX.

Figure 3 illustrates the construction of the plasmids carrying the vectors KTVIH5 (without SV-Neo) and KTVIH Neo (with SV-Neo).

Figure 4 illustrates construction of the plasmid carrying the vector MHMTK-Neo.

Figure 5 illustrates the construction of the plasmids carrying the fat-his (fat in sense direction) or atat (fat in antisense direction) vectors.

Figure 6 graphically depicts the preferential killing of PA317 cells infected with tathis vector (5 clones, TH1-5) compared to control PA317, upon infection with the three conditional lethal vectors shown and treatment with acyclovir (ACV).

Figure 7 depicts the construction of a viral vector carrying HIV inducible marker/reporter genes such as alkaline phosphatase (AP).

Figure 8 depicts the structure of an HIV inducible marker/reporter gene carried on a plasmid which can be transfected into cells.

Figure 9 graphically depicts a time course of HIV infection of Sup T1 cells carrying the AP marker in Figure 8 with HIV at various concentrations of AZT. The level of HIV infection was measured by taking small aliquots of supernatant.

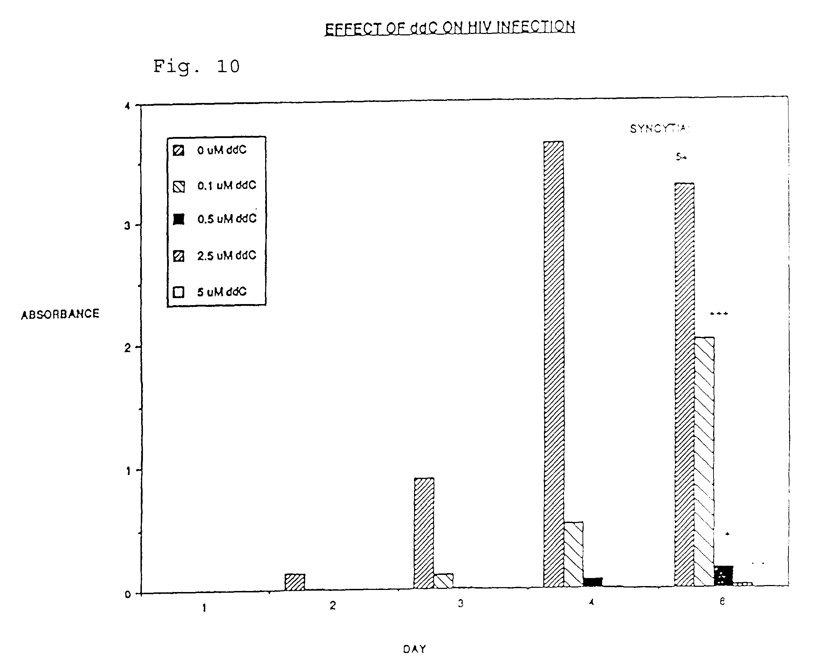

Figure 10 graphically depicts the results of the same experiment as in Figure 9, but with ddC as the HIV inhibitor.

Figure 11 diagrammatically illustrates the number of cells surviving after phleomycin selection upon transfection of cells with a plasmid which expresses the phlemoycin resistance gene (PRG) directly from a promoter (right) and with another which expresses PRG with a coding sequence interposed between it and the promoter.

Figure 12 depicts four plasmids designed to express retroviral proteins in mammalian cells. psvgp and pRSVenv are cotransfectd with a selectable marker, while pSVgp-DHFR and pRSVenv-phleo are the equivalent plasmids with the selectable marker placed downstream of the viral protein-coding regions.

Figure 13 depicts tnree sites of fusion of HIV env and MLV env after site-directed mutagenesis of both coding sequences to create new compatible restriction enzyme sites. The N terminal sequences are at the left in both cases. The numbering is according to nucleotide numbers. ST, SR, SE are the starts of fat, rev and env while TT, TR, and TE are the corresponding termination sites.

Figure 14 depicts the substitution of U3 in a 5' LTR by a heterologous promoter/enhancer which can be fused to either the Sac I, Bsst II or other site in the region.

Figure 15 illustrates a representative method for crossing transgenic mice expressing viral protein or vector RNA.

As used herein, "capable of inhibiting a function" means that the palliative either directly inhibits the function or indirectly does so, for example, by converting an agent present in the cells from one which would not normally inhibit a function of the pathogenic agent to one which does. Examples of such functions for viral diseases include absorption, replication, gene expression, assembly, and exit of the virus from infected cells. Examples of such functions for cancerous diseases include cell replication, susceptibility to external signals (e.g., contact inhibition), and lack of production of anti-oncogene proteins.

In one embodiment of the present invention, a gene capable of expressing Herpes Simplex virus thymidine kinase (HSVTK) is used to more effectively metabolize potentially antiviral nucleoside analogues, such as AZT or ddC. The HSVTK gene may be expressed under the control of a constitutive macrophage or T-cell-specific promoter and introduced into these cell types. AZT (and other nucleoside antivirals) must be metabolized by cellular mechanisms to the nucleotide triphosphate form in order to specifically inhibit retroviral reverse transcriptase and thus HIV replication (Furmam et al., Proc. Natl. Acad. Sci. USA 83:8333-8337, 1986). Constitutive expression of HSVTK (a nucleotide and nucleotide phosphate kinase with very broad substrate specificity) results in more effective metabolism of these drugs to their biologically active mucleotide triphosphate form. AZT or ddc therapy will thereby be more effective, allowing lower doses, less generalized toxicity, and higher potency against productive infection. Additional nucleoside analogues whose nucleotide triphosphate forms show selectivity for retroviral reverse transcriptase but, as a result of the substrate specificity of cellular nucleoside and nucleotide kinases are not phosphorylated, will be made more efficacious. A description of this method is set forth in Example 1.

### EXAMPLE 1

### Vectors Designed to Potentiate the Antiviral Effect of AZT and Analogues

I. All of the following retroviral vectors are based on the "N2" vector (see Keller et al., Nature 318:149-154, 1985). Consequently, 5' and 3' Eco R1 LTR fragments (2.8 and 1.0 kb, respectively) were initially subcloned into plasmids containing polylinkers (into SK+ to give pN2R5[+/=]; into pUC31 to give p31N2R5[+/-] and p31N2R3[+/-] to facilitate vector construction. In one case, a 1.2 kb Cla I/Eco R1 5' LTR fragment was subcloned into the same sites of an SK+ vector to give pN2C. In another case, the 5' LTR containing a 6 bp deletion of the splice donor sequence was subcloned as a 1.8 kb Eco R1 fragment into pUC31 (p31N25delta[+]). The coding region and transcriptional termination signals of HSV-1 thymidine kinase gene was isolated as a 1.8 kb Bgl II/Pvu II fragment from plasmid 322TK (3.5 kb Bam H1 fragment of HSVTK cloned into Bam H1 of pBR322) and cloned into Bgl II/Sma I-digested pUC31 (pUCTK). For constructs which require deletion of the terminator signals, pUCTK was digested with Sma I and Bam H1. The remaining coding sequences and sticky-end Bam H1 overhang were reconstituted with a double-stranded oligonucleotide made from the following oligomers: 
5′ GAG AGA TGG GGG AGG CTA ACT GAG 3′
and 5′ GAT CCT CAG TTA GCC TCC CCC ATC TCT C 3′
 forming the construct pTK delta A.

For diagnostic purposes, the oligos were designed to destroy the Sma I site while keeping its Ava I site without changing the translated protein.

The 0.6 kb HIV promoter sequences were cloned as a Dra I/Hind III fragment from pCV-1 (see Arya et al., Science 229:69-73, 1985) into Hinc II/Hind III-cut SK⁺ (SKHL).

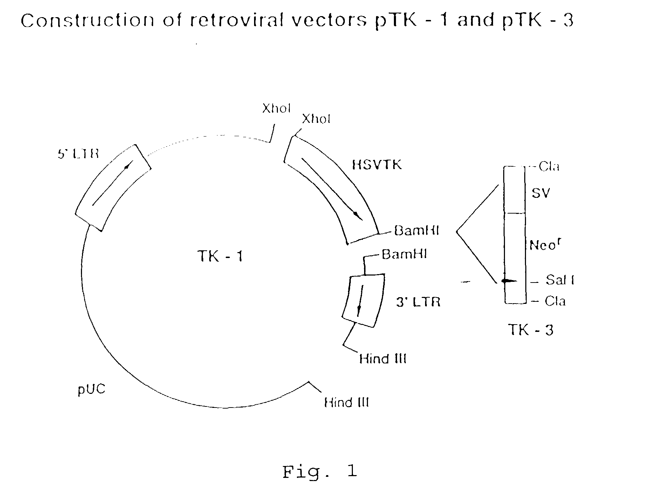

### II. Construction of TK-1 and TK-3 retroviral vectors (see Figure 1).

1. The 5 kb Xho I/Hind III 5' LTR and plasmid sequences were isolated from p31N2R5(+).
2. HSVTK coding sequences lacking transcriptional termination sequences were isolated as 1.2 kb Xho I/Bam H1 fragment from pTKdeltaA.
3. 3' LTR sequences were isolated as a 1.0 kb Bam H1/Hind III fragment from pN2R3(-).
4. The fragments from steps 1-3 were mixed, ligated, transformed into bacteria, and individual clones identified by restriction enzyme analysis (TK-1).
5. TK-3 was constructed by linearizing TK-1 with Bam H1, filling in the 5' overhang and blunt-end ligating a 5'-filled Cla I fragment containing the bacterial lac UV5 promoter, SV40 early promoter, plus Tn5 Neo^{r} gene. Kanamycin-resistant clones were isolated and individual clones were screened for the proper orientation by restriction enzyme analysis.

These constructs were used to generate infectious recombinant vector particles in conjunction with a packaging cell line, such as PA317, as described above.

Administration of these retroviral vectors to human T-cell and macrophage/monocyte cell lines can increase their resistance to HIV in the presence of AZT and ddc compared to the same cells without retroviral vector treatment.

Preparation, concentration and storage of the retroviral vector preparations would be as described above. Treatment would be as previously described but ex corpore treatment of patients' cells would aim for uninfected potentially susceptible T-cells or monocytes. One preferred method of targeting the susceptible cell is with vectors which carry HIV env or hybrid env (see Section headed "Cell Line Specific Retroviruses", below) to direct absorption of vector particles to CD4⁺ cells. Normal adults have about 5 x 10⁹ T4 cells in their total blood and about the same number of monocytes.

A preferred method of administration would be leukophoresis, where about 20% of an individual's PBLs can be removed at any one time and manipulated in vitro. Thus, about 2 x 10⁹ cells would be treated and replaced. Since the current maximum titres are around 10⁶/ml, this would require 2 to 20 liters of starting viral supernatant. Repeat treatments would be performed. Another route which may be used is the aspiration of bone marrow, infection with retroviral vector and return of this infected marrow (Gruber et al., Science 230:1057, 1985) to the patient. Since the marrow replication will amplify the vector expression through cell replication, doses in the range of 10⁵ - 10⁶/kg body weight can be used. Treatment with AZT would be at lower than normal levels to avoid toxic side effects, but still efficiently inhibit spread of HIV. The efficacy of the treatment can be assayed by measuring the usual indicators of disease progression, including antibody level, viral antigen production, infectious HIV levels, or levels of nonspecific infections.

Expression of the palliative via action of the enzyme expressed by a proviral vector of the invention should be limited by the presence of some intracellular signal identifying the pathogenic state in order to avoid destruction of nonpathogenic cells. This cell-type specificity may also be conferred at the level of infection by targeting recombinant retrovirus carrying the vector to cells having or being susceptible to the pathogenic condition.

A recombinant retrovirus of the invention (e.g a recombinant MLV retrovirus) may, for example, carry a vector construct containing an enzyme gene expressed from an event-specific promoter, such as a cell cycle-dependent promoter (e.g., human cellular thymidine kinase or transferrin receptor promoters), which will be transcriptionally active only in rapidly proliferating cells, such as tumors. In this manner, rapidly replicating cells, which contain factors capable of activating transcription from these promoters, are preferentially destroyed by the cytotoxic agent produced by the proviral construct.

In a second embodiment, the gene for the enzyme producing the cytotoxic agent is under the control of a tissue-specific promoter, where the tissue specificity corresponds to the origin of the tumor. Since the viral vector preferentially integrates into the genome of replicating cells (for example, normal liver cells are not replicating, while those of a hepatocarcinoma are), these two levels of specificity (viral integration/replication and tissue-specific transcriptional regulation) lead to preferential killing of tumor cells. Additionally, event-specific and tissue-specific promoter elements may be artificially combined such that the gene product responsible for production of the cytotoxic agent is expressed only in cell types satisfying both criteria (e.g., in the example above, combined promoter elements are functional only in rapidly dividing liver cells). Transcriptional control elements may also be amplified to increase the stringency of cell-type specificity.

These transcriptional promoter/enhancer elements need not necessarily be present as an internal promoter (lying between the viral LTRs) but may be added to or replace the transcriptional control elements in the viral LTRs which are themselves transcriptional promoters, such that condition-specific transcriptional expression will occur directly from the modified viral LTR. In this case, either the condition for maximal expression will need to be mimicked in retroviral packaging cell lines (e.g., by altering growth conditions, supplying necessary transregulators of expression or using the appropriate cell line as a parent for a packaging line), or the LTR modification is limited to the 3' LTR U3 region, to obtain maximal recombinant viral titers. In the latter case, after one round of infection/integration the 3' LTR U3 is now also the 5' LTR U3 giving the desired tissue specific expression.

In the case of a proviral vector construct according to the present invention carrying a HSVTK gene downstream and under the transcriptional control of an HIV promoter (which is known to be transcriptionally silent except when activated by HIV fat protein), expression of the tat gene product in human cells infected with HIV and carrying the proviral vector construct causes increased production of HSVTK. The cells (either in vitro or in vivo) are then exposed to a drug such as acyclovir or its analogues (FIAU, FIAC, DHPG). These drugs are known to be phosphorylated by HSVTK (but not by cellular thymidine kinase) to their corresponding active nucleotide triphosphate forms (see, for example, Schaeffer et al., Nature 272:583, 1978). Acyclovir and FIAU triphosphates inhibit cellular polymerases in general, leading to the specific destruction of cells expressing HSVTK in transgenic mice (see Borrelli et al., Proc. Natl. Acad. Sci. USA 85:7572, 1988). Those cells containing the recombinant vector and expressing HIV tat protein are selectively killed by the presence of a specific dose of these drugs. In addition, an extra level of specificity is achieved by including in the vector the HIV rev protein, responsive CRS/CAR sequences. In the presence of the CRS sequence gene expression is suppressed, except in the presence of the CAR sequences and the rev protein. Example 2 provides an illustration of this technique.

### EXAMPLE 2

### Vector to Conditionally Potentiate the Toxic Action of ACV or Its Analogues

### Construction of Vectors

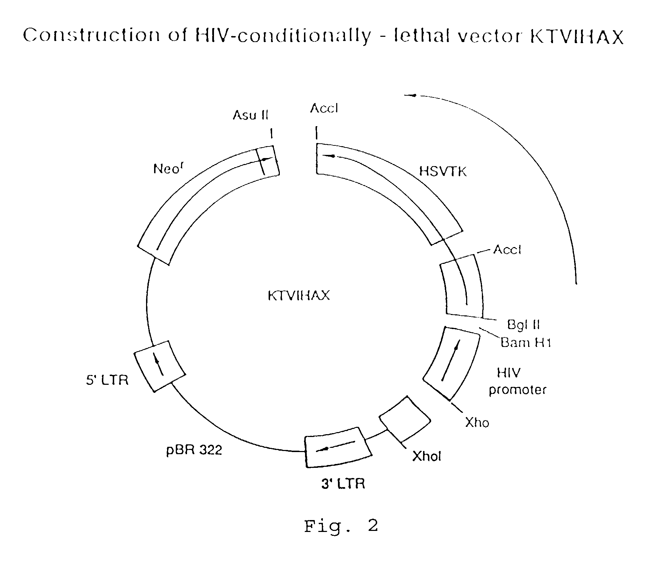

### A. Construction of pKTVIHAX (see Figure 2).

1. A 9.2 kb Asu II/Xho I fragment was isolated from vector pN2 DNA.
2. A 0.6 kb Xho I/Bam H1 promoter fragment was isolated from plasmid pSKHL.
3. A 0.3 kb Bg lII/Acc I and a 1.5 kb Acc I/Acc I fragment were purified from pUCTK.
4. The fragments from 1, 2, and 3 were ligated, transformed into bacteria, and appropriate Amp^{r} clones of the given structure identified by restriction enzyme analysis.

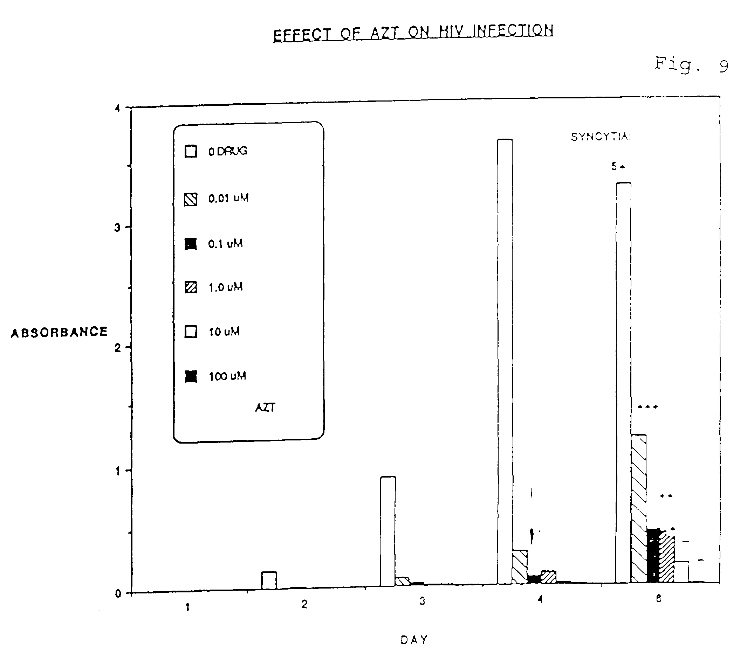

### B. Construction of pKTVIH-5 and pKTVIH5 Neo retroviral vectors (see Figure 8).

1. 4.5 kb 5' LTR and vector was isolated as an Xho I/Bam H1 fragment from vector p31N25delta(+).
2. The 1.0 kb 3' LTR was isolated as an Apa I/Bam H1 fragment from pN2R3(+) fragment.
3. The 0.6 kb HIV promoter element was isolated from pSKHL as an Apa I/Eco R1 fragment.
4. The HSVTK coding sequence and transcriptional termination sequences were isolated as 1.8 kb Eco Rl/Sal I fragment from pUCTK.
5. The fragments from 1-4 were combined, ligated, transformed into bacteria, and clones of the given structure were identified by restriction enzyme analysis (pKTVIH-5).
6. pKTVIH5 Neo was constructed by linearizing pKTVIH5 with Cla I; mixing with a 1.8 kb Cla I fragment containing the bacterial lac UV5 promoter, SV40 early promoter, and Tn5 Neo^{r} marker, ligating, transforming bacteria and selecting for kanamycin resistance. Clones with the insert in the indicated orientation were identitied by restriction analysis.

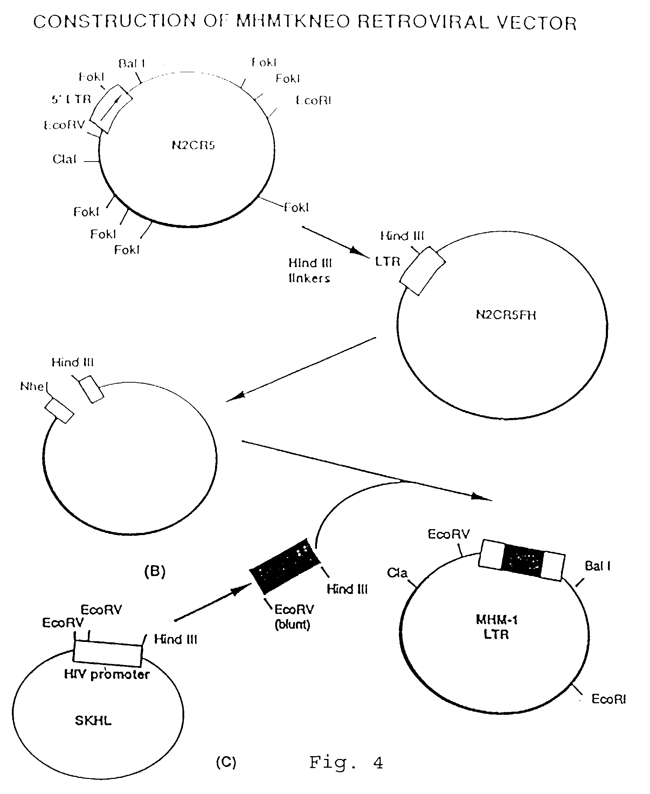

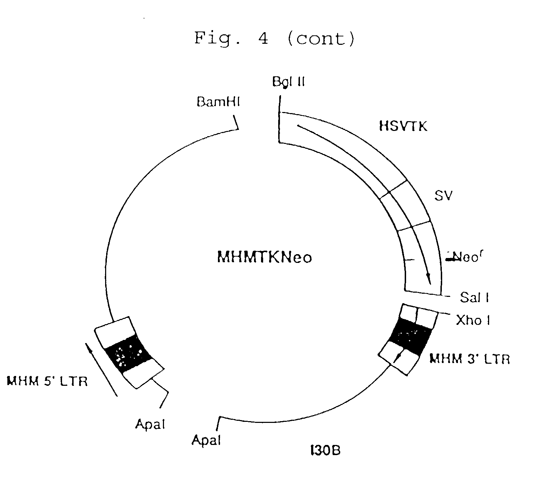

### C. Construction of MHMTK Neo retroviral vector (see Figure 4).

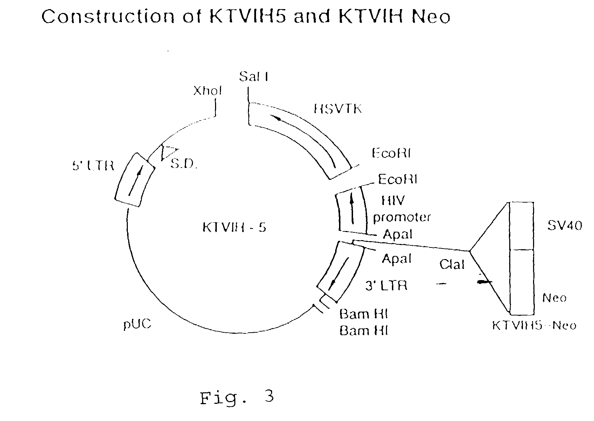

1 . Construction of intermediate plasmid MHM-1:
   a) plasmid pN2CR5 was linearized by partial digestion with Fok I, the 5' overhang filled in with deoxynucleotide triphosphates using Klenow DNA polymerase, and Hind III linkers inserted. After transformation into bacteria, a clone with a Hind III linker inserted in the MLV LTR Fok I site was identified by restriction enzyme analysis (pN2CR5FH).
   b) pN2CR5FH was linearized with Nhe I, the 5' overhang filled in with Klenow polymerase digested with Hind III, and the 4.3 kb fragment with promoterless MLV sequences isolated.
   c) 0.5 kb Eco RV/Hind III HIV promoter sequences were isolated from pSKHL.
   d) b and c were mixed, ligated, used to transform bacteria, and the structure of MHM-1 was confirmed by restriction enzyme analysis.
2. A 0.7 kb Eco RV/Bal I fragment isolated from MHM-1 was subcloned into the Eco RV site of plasmid I30B (a modified IBI30 plasmid containing additional Bgl II, Bst II, Neo I and Nde I sites in the polylinker). After transformation into bacteria, clones with the appropriate orientation were identified by restriction enzyme analysis (pMHMB).
3. pMHMB was digested with Apa I and Xho I and gel purified.
4. MHM-1 was digested with Apa I/Bam H1 and the 1.8 kb MHMLTR/leader sequence gel purified.
5. A 2.8 kb Bgl II/Sal I fragment containing the HSVTK coding region upstream of the SV40 early promoter driving Neo^{r} taken from pTK-3 (see Figure 3).
6. 3-5 were mixed, ligated, used to transform bacteria, anti appropriate clones were identified by restriction enzyme analysis.

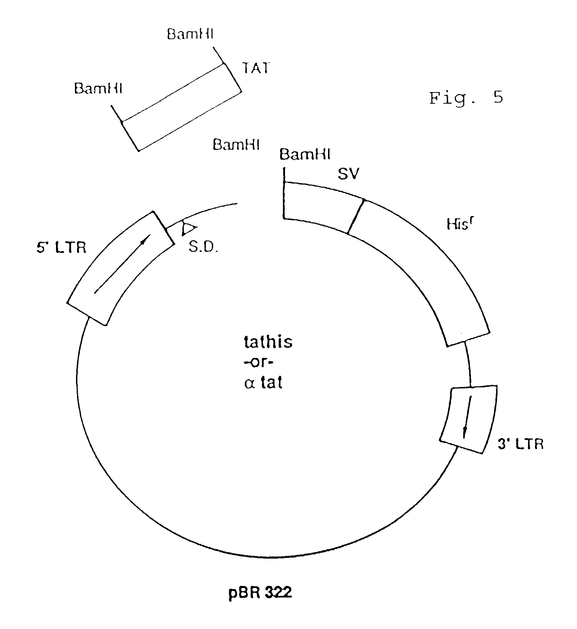

### D. Construction of tat and anti-tat expression vectors (see Figure 5).

These vectors are used as pseudo-HIV to test-activate fat-dependent HSVTK vectors.
1. The His^{r} expression vector pBamHis was linearized with Bam H1 and treated with calf intestinal phosphatase.
2. The Sac I site of pCV-1 was mutagenized to a Bam H1 site and the 350 bp Bam H1 coding sequence of HIV fat was isolated.
3. The fragments purified in steps 1 and 2 were mixed, ligated, used to transform bacteria, and clones with tat in both orientations were identified by restriction enzyme analysis.

These constructs were used to generate infectious recombinant vector particles in conjunction with a packaging cell line such as PA317, as described above.

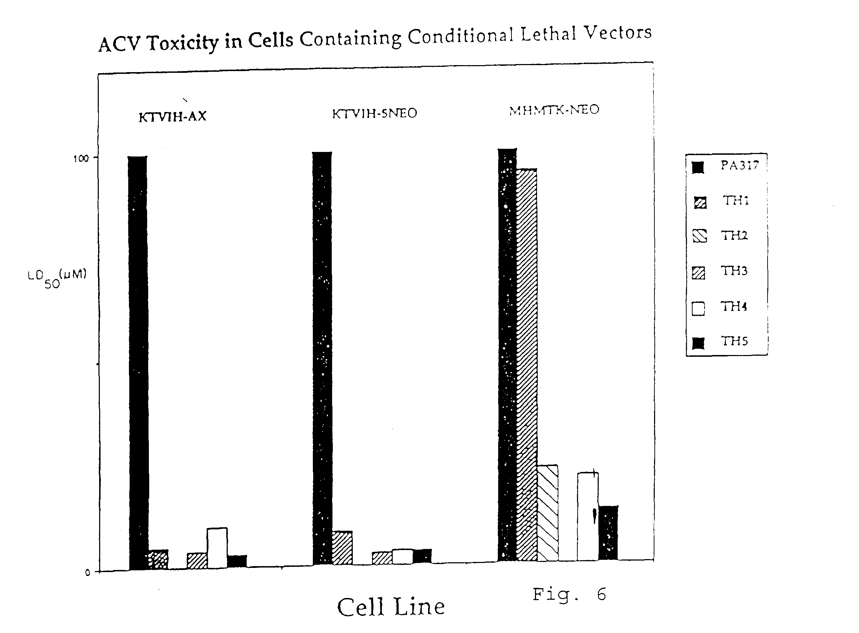

The biological properties of these retroviral vectors are described hereinafter. The HIV fat gene ("tathis" vector -- see Figure 5) was transfected into mouse PA317 cells. Five individual histidinol-resistant subclones were obtained (TH 1-5) which express HIV fat. These cells are thus an experimental model for HIV infection. The vectors KTVIHAX, KTVIH5NEO, and MHMTKNEO, were subsequently introduced by infection into these fat-expressing cell lines as well as their parent cell line lacking fat. Cell viability was then determined in various concentrations of the HSVTK-specific cytotoxic drug, acyclovir. The data are reported here as LD₅₀ (the drug concentration at which 50% toxicity is observed). The parental cell line containing the vector but lacking fat (non-HIV-infected model) showed no detectable toxicity by ACV at the concentrations tested (see Figure 6). These cells thus require 100 uM ACV or greater for cytotoxicity. This is true also for these cells lacking the vectors. Thus the vectors alone, ACV alone, or even the vector +ACV (solid boxes) is not cytotoxic. However, cell lines which express HIV tat (the experimental representation of an HIV infection) are effectively killed by ACV. This is true to varying degrees for all three vectors tested. These data indicate that HIV-infected cells will be killed in the presence of ACV and "potentiator" vectors.

Similarly, HIV infection of human T-cell lines 4/- FIAU showed preferential inhibition (through cell killing) of HIV infection. Cultures were first treated with vector, then challenged with low multiplicity HIV infection for 4 days. Viral supernatants were titred using the HIV, as described in Example 3.

In the case of HIV-infected cells, expression of the conditionally lethal HSVTK gene was made even more HIV specific by including cis-acting elements in the transcript ("CRS/CAR"), which require an additional HIV gene product, rev, for optimal activity (Rosen et al., Proc. Natl. Acad. Sci. USA 85:2071, 1988). More generally, cis elements present in mRNAs have been shown in some cases to regulate mRNA stability or translatability. Sequences of this type (i.e., post-transcriptional regulation of gene expression) may be used for event- or tissue-specific regulation of vector gene expression. In addition, multimerization of these sequences (i.e., rev-responsive "CRS/CAR" or tat-responsive "TAR" elements for HIV) could result in even greater specificity. It should be noted that this kind of conditional activation of an inactive precursor into an active product in cells may also be achieved using other viral vectors with a shorter term effect, e.g., adenovirus vectors.

Production, concentration and storage of vector preparations, is as previously described. Administration is by direct in vivo administration as before or by ex corpore treatment of PBL and or bone marrow. Doses will be at approximately the same levels as for Example 1. Targeting of viral vector infection will not be through the CD4 receptor, but may be accomplished through making vector particles with hybrid MLVenv-CD4 "envelope" proteins (see below) to target gp 120 expressing cells (i.e., those infected with HIV). This inversion of the normal virus-receptor interaction in order to target virally infected cells can be used with all types of viruses.

In a similar manner to the preceding embodiment, the retroviral vector construct can carry a gene for phosphorylation, phosphoribosylation, ribosylation, or other metabolism of a purine- or pyrimidine-based drug. This gene may have no equivalent in mammalian cells and might come from organisms such as a virus, bacterium, fungus, or protozoan. An example of this would be the E. coli guanine phosphoribosyl transferase gene product, which is lethal in the presence of thioxanthine (see Besnard et al., Mol. Cell. Biol. 7:4139-4141, 1987). Conditionally lethal gene products of this type have potential application to many presently known purine- or pyrimidine-based anticancer drugs, which often require intracellular ribosylation or phosphorylation in order to become effective cytotoxic agents. The conditionally lethal gene product could also metabolize a nontoxic drug, which is not a purine or pyrimidine analogue, to a cytoxic form.

Mammalian viruses in general tend to have "immediate early" genes which are necessary for subsequent transcriptional activation from other viral promoter elements. Gene products of this nature are excellent candidates for intracellular signals (or "identifying agents") of viral infection. Thus, conditionally lethal genes from transcriptional promoter elements responsive to these viral "immediate early" gene products could specifically kill cells infected with any particular virus. Additionally, since the human α and β interferon promoter elements are transcriptionally activated in response to infection by a wide variety of nonrelated viruses, the introduction of vectors expressing a conditionally lethal gene product like HSVTK, for example, from these viral-responsive elements (VRE) could result in the destruction of cells infected with a variety of different viruses.

### Example 3

### Expression of Markers

The above-described technique of expressing a palliative in a cell, in response to some identifying agent, can also be modified to enable detection of a particular gene in a cell which expresses an identifying protein (for example, a gene carried by a particular virus), and hence enable detection of cells carrying that virus. In addition, this technique enables the detection of viruses (such as HIV) in a clinical sample of cells carrying an identifying protein associated with the virus.

This modification can be accomplished by providing a genome coding for a product, the presence of which can be readily identified (the "marker product"), and carrying a promoter, which responds to the presence of the identifying protein in indicator cells, by switching expression of the reporting product between expressing and nonexpressing states. For example, HIV, when it infects suitable indicator cells, makes fat and rev. The indicator cells can thus be provided with a genome (such as by infection with an appropriate recombinant retrovirus) which codes for a marker gene, such as the alkaline phosphatase gene, β-galactosidase gene or the luciferase gene, and a promoter, such as the HIV promoter, which controls expression of the marker gene. When the indicator cells are exposed to a clinical sample to be tested, and the sample-contains HIV, the indicator cells become infected with HIV, resulting in fat and/or rev expression (an identifying protein) therein. The HIV expression controls in the indicator cells would then respond to fat and/or rev proteins by switching expression of genes encoding β-galactosidase, luciferase, or alkaline phosphatase (marker products) from normally "off" to "on." In the case of β-galactosidase or alkaline phosphatase, exposing the cells to substrate analogues results in a color or fluorescence change if the sample is positive for HIV. In the case of luciferase, exposing the sample to luciferin will result in luminescence if the sample is positive for HIV. For intracellular enzymes such as β-galactosidase, the viral titre can be measured directly by counting colored or fluorescent cells, or by making cell extracts and performing a suitable assay. For secreted enzymes, such as an engineered form of alkaline phosphatase, small samples of culture supernatant are assayed for activity, allowing continuous monitoring of a single culture over time. Thus, different forms of this marker system can be used for different purposes. These include counting active virus or sensitively and simply measuring viral spread in a culture and the inhibition of this spread by various drugs.

Further specificity can be incorporated into the preceding system by testing for the presence of the virus either with or without neutralizing antibodies to that virus. For example, in one portion of the clinical sample being tested, neutralizing antibodies to HIV may be present; whereas in another portion there would be no neutralizing antibodies. If the tests were negative in the system where there were antibodies and positive where there were no antibodies, this would assist in confirming the presence of HIV.

Within an analogous system for an in vitro assay, the presence of a particular gene, such as a viral gene, may be determined in a cell sample. In this case, the cells of the sample are infected with a suitable retroviral vector which carries the reporter gene linked to the expression controls of the virus of interest. The reporter gene, after entering the sample cells, will express its reporting product (such as β-galactosidase or luciferase) only if the host cell expresses the appropriate viral proteins.

These assays are more rapid and sensitive, since the reporter gene can express a greater amount of reporting product than identifying agent present, which results in an amplification effect. Example 4 describes a representative technique for detecting a gene which expresses an identifying protein.

### EXAMPLE 4

### HIV-Specific Marker System or Assay

### A. Constructs

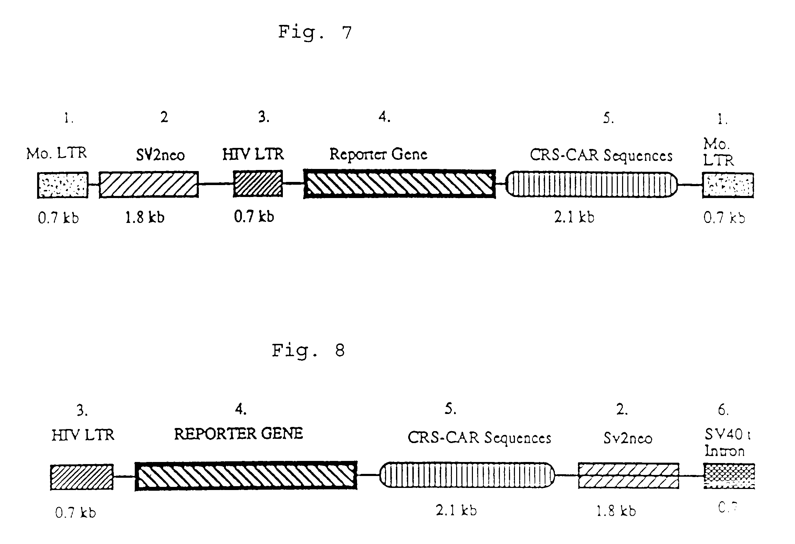

Reporter constructs under the control of the HIV expression system are shown in Figure 7 (a recombinant retroviral vector) and in Figure 8 (a simple plasmid used by transfection) . The pieces of these preferred vector and plasmid reporters were derived as follows:

The retroviral backbone was derived from the construct pAFVXM (Krieger et al., Cell 38:384, 1984), which had been linearized using Xho I and Cla I. SV₂neo was obtained from the plasmid pKoneo (Hanahen, unpubl.) by isolation of the 1.8 kb Cla I fragment.

The HIV LTR was isolated as a 0.7 kb Hind III fragment from the plasmid pc15CAT (Arya et al., Science 229:69, 1985). Beta-gal was obtained from the plasmid pSP65 β-gal (Cepko, pers. comm.) as a Hind III-Sma I fragment. A secreted form of human placental alkaline phosphatase was produced by introduction of a universal terminator sequence after amino-acid 489 of the cell surface form of alkaline phosphatase (as described by Berger et al., Gene 66:1, 1988). The secreted alkaline phosphatase gene was isolated as a 1.8 kb Hind III to Kpn I fragment. The CRS-CAR sequences from HIV env were obtained by isolating the 2.1 kb Kpn I to Bam H1 fragment from HTLVIIIB/BH10R3 (Fisher et al., Science 233:655, 1986). This fragment was inserted into pUC31 linearized by Bam HI, and Kpn I pUC31 is pUC19 (Yanisch-Perron et al., Gene 33:103, 1985) with extra Xho I, B-gl II, Bssh II and Nco I sites between the Eco R1 and Kpn I sites of pUC19. The Bam H1 site of the resulting construct was converted to a Nco I site to allow resection of the CRS-CAR sequences by Nco I digestion. The SV40 t intron was obtained from pSVOL (de Wet et al., Mol. Cell. Biol. 7:725, 1987) as a 0.8 kb Nco I to Bam H1 fragment.

### B. Indicator Cells and Retroviral Vectors

Human T-cell (H-9, CEM and Sup T1) and monocyte (U-937) cell lines were obtained from ATCC, and maintained in RPM1 1640 medium supplemented with 10% fetal bovine serum and 1% penicillin/streptomycin.

The nonretroviral vectors were introduced into cell lines by electroporation using a Bio-Rad Gene Pulser. The cell lines were selected in G-418 (1 mg/ml) for 2-3 weeks to obtain stable G-418_{R} cell lines, and then dilution cloned to obtain clonal cell lines.

The pAF vectors were transfected into the PA317 packaging cell line as a calcium phosphate precipitate (Wigler et al., Cell 16:777, 1979). The virus-producing PA317 cells were co-cultivated with human monocyte cell lines for 24 hours in the presence of polybrene, after which the suspension cells were removed and selected in G-418 and subcloned as above.

### C. Assay

Stable cell lines were infected with HIV (HTLV III_{B}) and the cells (β-gal) or media (alkaline phosphatase) assayed on a daily basis for 6 days post-infection.

### β-Galactosidase Assay

Infected cells could be assayed by either: (i) In situ histochemical staining as described by MacGregor et al. Somatic Cell and Mol. Genetics 13:253, 1987); or (ii) by using cell extracts in a solution enzymatic assay with ONPG as a substrate (Norton and Coffin, Mol. Cel. Biol. 5:281, 1985).

### Soluble Alkaline Phosphatase Assay

Medium was removed from infected cells, microfuged for 10 seconds, and then heated to 68°C for 10 minutes to destroy endogenous phosphatases. The medium was then microfuqed for 2 minutes and an aliquot (10-50 µl) removed for assay. 100 µl of buffer (1 M diethanolamine, pH 9.8; 0.5 Mm MgCl₂; 10 mM L-homoarginine) was added and then 20 µl of 120 mM p-nitrophenylphosphate (in buffers) was added. The A₄₀₅ of the reaction mixture was monitored using an automatic plate reader.

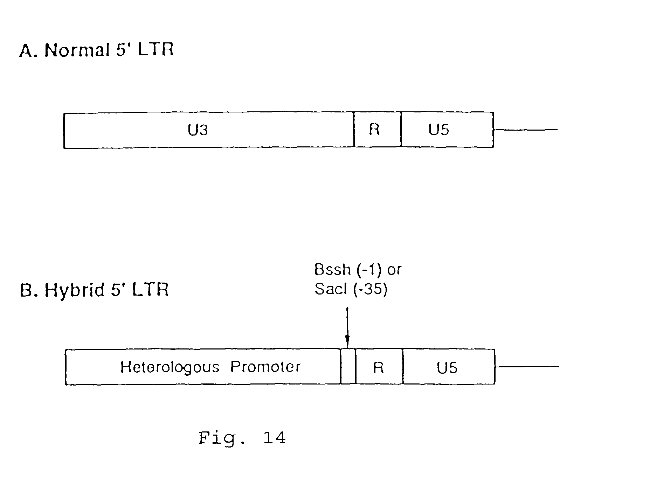

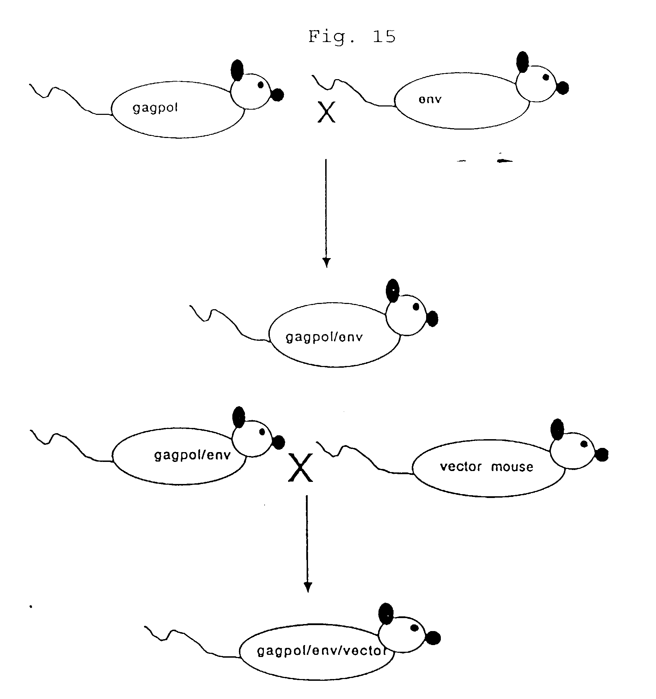

Figures 14 and 15 depict typical results of a time course of infection of Sup T1 cells using the alkaline phosphatase assay in the presence of varying concentrations of antiviral drugs. The "+" and "-" on day 6 indicate the presence or absence of syncytia.

The present invention provides a number of other techniques (described below) which can be used with the retroviral vector systems employed above, so as to enhance their performance. Alternatively, these techniques may be used with other gene-delivery systems.

### Example 5

### Packaging Cell Selection

Basically, at least five factors may be postulated as causes for low recombinant virus titres:
1. the limited availability of viral packaging proteins;
2. the limited availability of retroviral vector RNA genomes;
3. the limited availability of cell membrane for budding of the recombinant retroviruses;
4. the limited intrinsic packaging efficiency of the retroviral vector genome; and
5. the density of the receptir specific for the envelope of a given retrovirus.

As noted above, the limited availability of viral packaging proteins is the initial limiting factor in recombinant retrovirus production from packaging cells. When the level of packaging protein in the packaging cells is increased, titre increases to about 10⁵ infectious units/milliliter, following which increasing packaging protein level has no further effect on titres. However, titres can be further augmented by also increasing the level of retroviral vector genome available for packaging. Thus, as described herein, it is advantageous to select producer cells that manufacture the maximum levels of packaging proteins and retroviral vector genomes. It has been discovered that the methods of identifying, and thus selecting, packaging cells and producer cells, described earlier under the section entitled "Background of the Invention," tend to lead to selection of many producer cells which produce low titres for the reasons described below.

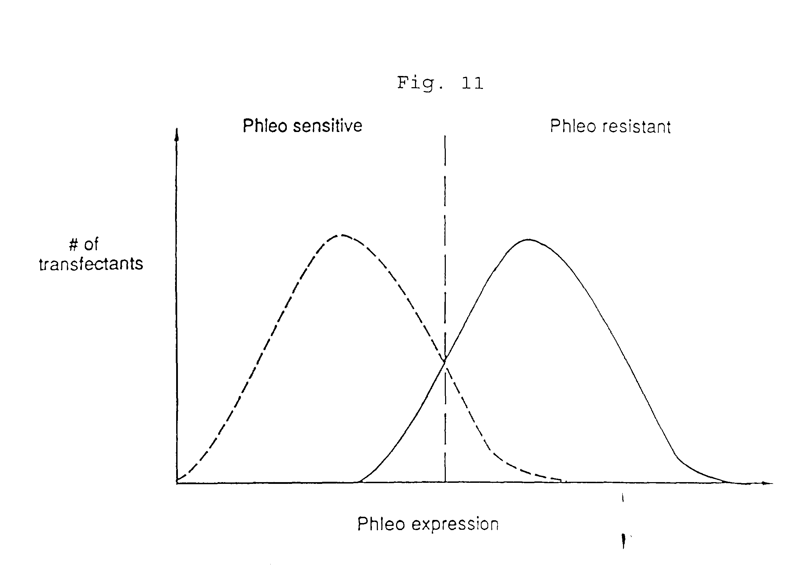

In obtaining packaging cell lines, advantage may be taken of the previously disadvantageous fact that the protein expression level of a gene downstream from the 5' LTR or other promoter, and spaced therefrom by an intervening gene, is substantially less than if the intervening gene were absent. Thus, the selectable gene is placed downstream from a gene of the packaging genome or the gene of interest carried by the vector construct, but is still transcribed under the control of the viral 5' LTR or other promoter without any splice donor or splice acceptor sites. This accomplishes two things. First, since the packaging genes or genes of interest are now upstream with no intervening gene between themselves and the promoter, their corresponding proteins (packaging protein or protein of interest) will be expressed at a higher level (5- to 20-fold) than the selectable protein. Second, the selectable protein will be expressed on average at a lower level, with the distribution of level of expression shifting toward lower levels. In the case of the phleo^{r} protein, this shift in distribution is illustrated by the broken curve indicated in Figure 11. However, the selection level for resistance to phleomycin remains the same, so that only the top-end expressing cells survive. The levels of the packaging protein or of the protein of interest will still be proportional, only in this case, a higher level of selectable protein corresponds to a much higher level of packaging protein or protein of interest.

Preferably, the foregoing procedure is performed using a plasmid carrying one of the proviral gag/pol or env packaging genes, along with a first selectable gene. These cells are then screened for the cells producing the highest levels of protein by reaction with an antibody against env (or possibly gag/pol), a second fluorescent antibody, and then sorted on a fluorescence-activated cell sorter (FACS). Alternatively, other tests for protein level may be used. Subsequently, the procedure and screening are repeated using those selected cells, and the other of the gag/pol or env packaging genes. In this step, a second selectable gene (different from the first) would be required downstream from the packaging gene and the cells producing the largest amount of the second viral protein selected. The procedure and screening are then repeated using the surviving cells, with a plasmid carrying the proviral vector construct bearing the gene of interest and a third selectable gene, different from the first or second selectable gene. As a result of this procedure, cells producing high titres of the desired recombinant retrovirus will be selected, and these can be cultured as required to supply recombinant retrovirus. In addition, gag and pol can be independently introduced and selected for.

Example 6 describes the construction of gag/pol and env plasmids designed to use these procedures.

### EXAMPLE 6

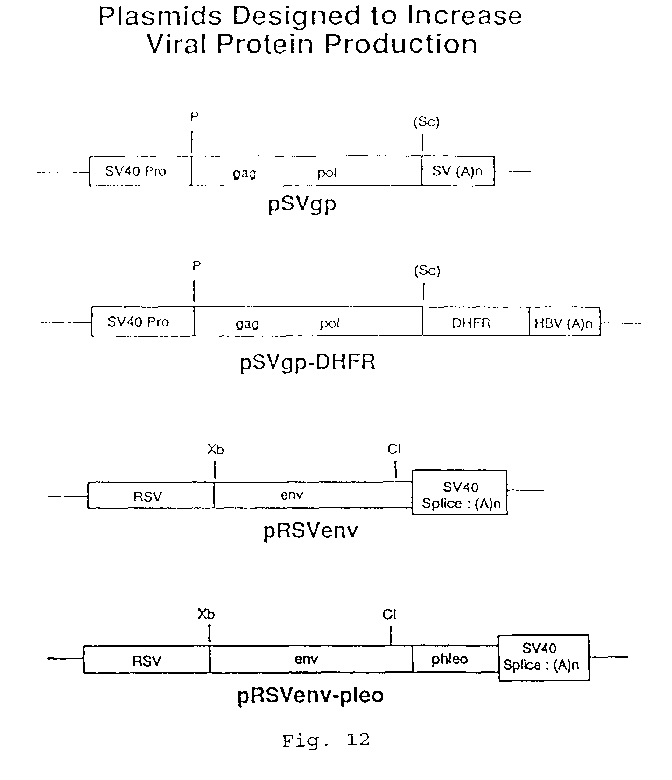

### Plasmids Designed to Make High Levels of Packaging Proteins (Figure 12)

1. The 2.7 kb Xba I fragment from pPAM (Miller et al., Mol. Cell. Biol. 5:431, 1985), which contains the amphotropic env segment, was cloned in pUC18 at the Xba I site, then removed with Hind III and Sma I. This fragment was cloned into the vector pRSV neo (Gorman et al., Mol. Cell. Biol. 2:1044, 1982; Southern et al., J. Mol. Appl. Genet. 1:327, 1982) cut with Hind III and Pvu II, to give pRSV env. A 0.7 kb Bam H1 to BstE II fragment from the plasmid pUT507 (Mulsant et al., Somat. Cell. Mol. Genet. 14:243, 1988) with the BstE II end filled in carries the phleo resistance coding sequence. The 4.2 kb Bam H1 to Xho I fragment, the contiguous 1.6 kb Xho I to Xba I (Xba I filled in) from RSVenv, and the phleo fragment were ligated to give pRSVenv-phleo.
2. A fragment from the Pst I site at nucleotide 563 of MLY (RNA Tumor Viruses, Vol. II, 1985, Cold Spring Harbor) to the Sca I site at 5870 was derived from pMLV-K (Miller et al., 1985, op. cit.) and cloned in the Pst I to Bam H1 (Bam H1 filled-in) fragment from p4aA8 (Jolly et al., Proc. Natl. Acad. Sci. USA 80:477, 1983) that has the SV40 promoter, the pBR322 ampicillin resistance and origin of replication and the SV40 poly A site. This gives pSVgp. pSVgpDHFR was made using the following fragments: the 3.6 kb Hind III to Sal I fragment from pSVgp containing the SV40 promoter plus MLY gag and some pol sequences; the 2.1 kb Sal I to Sca I fragment from pMI,V-K with the rest of the pol gene, the 3.2 kb Xba I (Xba I filled-in) to Pst I fragment from pF400 with the DHFR gene plus poly A site, pBR322 origin and half the ampicillin resistance gene; the 0.7 kb Pst I to Hind III fragment from pBR322 with the other half of the ampicillin resistance gene. This gives pSVgp-DHFR. All these constructs are shown in Figure 12. These plasmids can be transfected into 3T3 cells or other cells and high levels of gag, pol or env obtained.

An additional method for accomplishing selection is to use a gene selection in one round and its antisense in a subsequent round. For example, gag/pol may be introduced into an HPRT-deficient cell with the HPRT gene and selected for the presence of this gene using that media which requires HPRT for the salvage of purines. In the next round, the antisense to HPRT could be delivered downstream to env and the cell selected in 6 thioguanine for the HPRT-deficient phenotype. Large amounts of antisense HPRT would be required in order to inactivate the HPRT gene transcripts, assuming no reversion occurred.

### Envelope Substitutions

The ability to express gag pol and env functions separately allows for manipulation of these functions independently. A cell line that expresses ample amounts of gagpol can be used, for example, to address questions of titre with regard to env. One factor resulting in low titres is the density of appropriate receptor molecules on the target cell or tissue. Given that env expression is from a separate unit, a variety of envelope genes (requiring different receptor proteins), such as xenotropic, polytropic, or amphotropic envs from a variety of sources, can be tested for highest titres on a specific target tissue. Furthermore, envelopes from nonmurine retrovirus sources can be used for pseudotyping a vector. In addition, hybrid envelopes (as described below) can be used in this system as well, to tailor the tropism (and effectively increase titres) of a retroviral vector. Conversely, a cell line that expresses ample amounts of a given envelope gene can be employed to address questions of titre with regard to gag and pol.

### Alternative Viral Vector Packaging Techniques

Two additional alternative systems can be used to produce recombinant retroviruses carrying the vector construct. Each of these systems takes advantage of the fact that the insect virus, baculovirus, and the mammalian viruses, vaccinia and adenovirus, have been adapted recently to make large amounts of any given protein for which the gene has been cloned. For example, see Smith et al. (Mol. Cell. Biol. 3:12, 1983); Piccini et al. (Meth. Enzymology, 153:545, 1987); and Mansour et al. (Proc. Natl. Acad. Sci. USA 82:1359, 1985).

These viral vectors can be used to produce proteins in tissue culture cells by insertion of appropriate genes into the viral vector and, hence, could be adapted to make retroviral vector particles.

Adenovirus vectors are derived from nuclear replicating viruses and can be defective. Genes can be inserted into vectors and used to express proteins in mammalian cells either by in vitro construction (Ballay et al., EMBO J. 4:3861, 1985) or by recombination in cells (Thummel et al., J. Mol. Appl. Genetics 1:435, 1982).

One preferred method is to construct plasmids using the adenovirus Major Late Promoter (MLP) driving: (1) gag/pol, (2) env, (3) a modified viral vector construct. A modified viral vector construct is possible because the U3 region of the 5' LTR, which contains the viral vector promoter, can be replaced by other promoter sequences (see, for example, Hartman, Nucl. Acids Res. 16:9345, 1988). This portion will be replaced after one round of reverse transcriptase by the U3 from the 3' LTR.

These plasmids can then be used to make adenovirus genomes in vitro (Ballay et al., op. cit.), and these transfected in 293 cells (a human cell line making adenovirus E1A protein), for which the adenoviral vectors are defective, to yield pure stocks of gag, pol, env and retroviral vector carried separately in defective adenovirus vectors. Since the titres of such vectors are typically 10⁷-10¹¹/ml, these stocks can be used to infect tissue culture cells simultaneously at high multiplicity. The cells will then be programmed to produce retroviral proteins and retroviral vector genomes at high levels. Since the adenovirus vectors are defective, no large amounts of direct cell lysis will occur and retroviral vectors can be harvested from the cell supernatants.

In an alternative system (which is more truly extracellular), the following components are used:
1. gag/pol and env proteins made in the baculovirus system in a similar manner as described in Smith et al. (supra) (or in other protein production systems, such as yeast or E. coli);
2. viral vector RNA made in the known T7 or SP6 or other in vitro RNA-generating system (see, for example, Flamant and Sorge, J. Virol. 62:1827, 1988);
3. tRNA made as in (2) or purified from yeast or mammalian tissue culture cells;
4. liposomes (with embedded env protein); and
5. cell extract or purified necessary components (when identified) (typically from mouse cells) to provide env processing, and any or other necessary cell-derived functions.

Within this procedure (1), (2) and (3) are mixed, and then env protein, cell extract and preliposome mix (lipid in a suitable solvent) added. It may, however, be necessary to earlier embed the env protein in the liposomes prior to adding the resulting liposome-embedded env to the mixture of (1), (2), and (3). The mix is treated (e.g., by sonication, temperature manipulation, or rotary dialysis) to allow encapsidation of the nascent viral particles with lipid plus embedded env protein in a manner similar to that for liposome encapsidation of pharmaceuticals, as described in Gould-Fogerite et al., Anal. Biochem. 148:15, 1985). This procedure allows the production of high titres of replication incompetent recombinant retroviruses without contamination with pathogenic retroviruses or replication-competent retroviruses.

### Cell Line-Specific Retroviruses - "Hybrid Envelope"

The host cell range specificity of a retrovirus is determined in part by the env gene products. For example, Coffin, J. (RNA Tumor Viruses 2:25-27, Cold Spring Harbor, 1985) notes that the extracellular component of the proteins from murine leukemia virus (MLV) and Rous Sarcoma virus (RSV) are responsible for specific receptor binding. The cytoplasmic domain of envelope proteins, on the other hand, are understood to play a role in virion formation. While pseudotyping (i.e., the encapsidation of viral RNA from one species by viral proteins of another species) does occur at a low frequency, the envelope protein has some specificity for virion formation of a given retrovirus. By creating a hybrid env gene product (i.e., specifically, an env protein having cytoplasmic regions and exogenous binding regions which are not in the same protein molecule in nature) the host range specificity may be changed independently from the cytoplasmic function. Thus, recombinant retroviruses can be product which will specifically bind to preselected target cells.

In order to make a hybrid protein in which the receptor binding component and the cytoplasmic component are from different retroviruses, a preferred location for recombination is within the membrane-spanning region of the cytoplasmic component. Example 7 describes the construction of a hybrid env gene which expresses a protein with the CD4 binding portion of the HIV envelope protein coupled to the cytoplasmic domain of the MLV envelope protein.

### EXAMPLE 7

### Hybrid HIV-MLV Envelopes

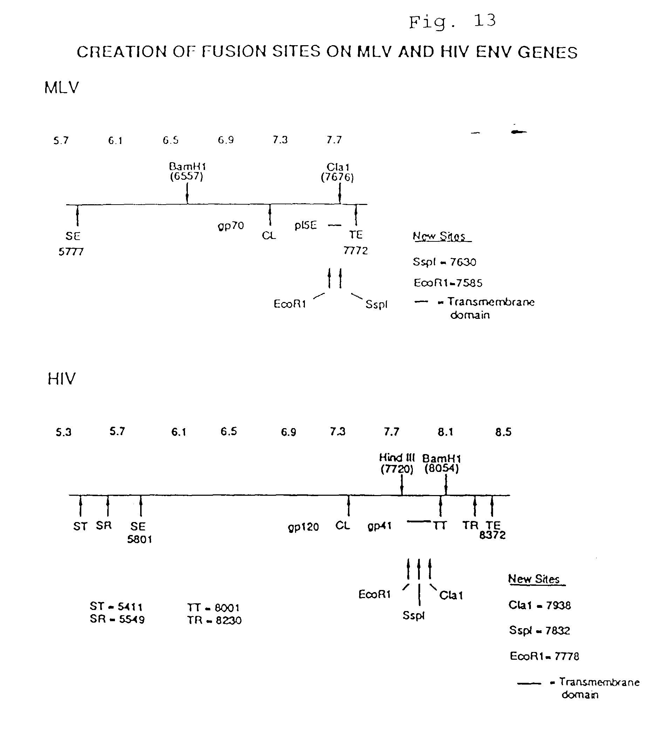

A hybrid envelope gene is prepared using in vitro mutagenesis (Kunkel, Proc. Natl. Acad. Sci. USA 82:488-492, 1985) to introduce a new restriction site at an appropriate point of junction. Alternatively, if the two envelope sequences are on the same plasmid, they can be joined directly at any desired point using in vitro mutagenesis. The end result in either case is a hybrid gene containing the 5' end of the HIV gp 160 and the 3' end of MLB p15E. The hybrid protein expressed by the resulting recombinant gene is illustrated in Figure 13 and contains the HIV gp120 (CD4 receptor binding protein), the extracellular portion of HIV gp 41 (the gp 120 binding and fusigenic regions), and the cytoplasmic portion of MLV p15E, with the joint occurring at any of several points within the host membrane. A hybrid gene made by fusion at the new Eco R1 site shown in Figure 13 is expressed under control of the CMVIE gene in human Sup T1 cells and leads to syncytia, as is the case with the authentic HIV env gene. Thus the hybrid is correctly transported to the cell surface and displayed there.

While Example 7 illustrates one hybrid protein produced from two different retroviruses, the possibilities are not limited to retroviruses or even simply other viruses. For example, the beta-receptor portion of human interleukin-2 may be combined with the envelope protein of MLV. In this case, a recombination would preferably be located in the gp 70 portion of the MLV env gene, leaving an intact p15E protein. Furthermore, the foregoing technique may be used to create a recombinant retrovirus with an envelope protein which recognizes antibody Fc segments. Monoclonal antibodies which recognize only preselected target cells only could then be bound to such a recombinant retrovirus exhibiting such envelope proteins so that the retrovirus would bind to and infect only those preselected target cells.

### Site-Specific Integration

Targeting a retroviral vector to a predetermined locus on a chromosome increases the benefits of gene-delivery systems. A measure of safety is gained by direct integration to a "safe" spot on a chromosome, i.e., one that is proven to have no deleterious effects from the insertion of a vector. Another potential benefit is the ability to direct a gene to an "open" region of a chromosome, where its expression would be maximized. Two techniques for integrating retroviruses at specific sites are described below.

### (i) Homologous Recombination

One technique for integrating an exogenous gene of a vector construct of a recombinant retrovirus into a specific site in a target cell's DNA employs homologous recombination. Plasmids containing sequences of DNA of greater than about 300 bp that are homologous to genomic sequences have been shown to interact (either by replacement or insertion) with those genomic sequences at a rate that is greater than 10³-fold over a specific interaction in the absence of such homology (see Thomas and Capecchi, Cell 51:503-12, 1987; and Doetscheman et al., Nature 330:576-78, 1987). It has been shown that an insertion event, or alternatively, a replacement event, may be driven by the specific design of the vector.

In order to employ homologous recombination in site-specific retroviral integration, a vector construct should be modified such that (a) homologous sequences (to the target cell's genome) are incorporated into the construct at an appropriate location; and (b) the normal mechanism of integration does not interfere with the targeting occurring due to homologous sequences. A preferred approach in this regard is to add homologous sequences (greater than about 300 bp) in the 3' LTR, downstream from the U3 inverted repeat. In this approach, the construct is initially made with a region of homology inserted in the 3' LTR at the Nhe 1 site in U3. Reverse transcription in the host cell will result in a duplication of the region of homology in the 5' LTR within 31 bp of the end of the inverted repeat (IR). Integration into the host genome will occur in the presence or absence of the normal integration mechanism. The gene in the vector may be expressed, whether from the LTR or from an internal promoter. This approach has the effect of placing a region of homology near a potential free end of the double-stranded retrovirus vector genome. Free ends are known to increase the frequency of homologous recombination by a factor of approximately 10. In this approach, it may be necessary to defeat the normal mechanism of integration, or to at least modify it to slow down the process, allowing time for homologous DNAs to line up. Whether this latter modification is required in a particular case can be readily ascertained by one skilled in the art.

### (ii) Integrase Modification

Another technique for integrating a vector construct into specific, preselected sites of a target cell's genome involves integrase modification.

The retrovirus pol gene product is generally processed into four parts: (i) a protease which processes the viral gag and pol products; (ii) the reverse transcriptase; and (iii) RNase H, which degrades RNA of an RNA/DNA duplex; and (iv) the endonuclease or "integrase."

The general integrase structure has been analyzed by Johnson et al. (Proc. Natl. Acad. Sci. USA 83:7648-7652, 1986). It has been proposed that this protein has a zinc binding finger with which it interacts with the host DNA before integrating the retroviral sequences.

In other proteins, such "fingers" allow the protein to bind to DNA at particular sequences. One illustrative example is the steroid receptors. In this case, one can make the estrogen receptor, responding to estrogens, have the effect of a glucocorticoid receptor, responding to glucocorticoids, simply by substituting the glucocorticoid receptor "finger" (i.e., DNA binding segment) in place of the estrogen receptor finger segment in the estrogen receptor gene. In this example, the position in the genome to which the proteins are targeted has been changed. Such directing sequences can also be substituted into the integrase gene in place of the present zinc finger. For instance, the segment coding for the DNA binding region of the human estrogen receptor gene may be substituted in place of the DNA binding region of the integrase in a packaging genome. Initially, specific integration would be tested by means of an in vitro integration system (Brown et al., Cell 29:347-356, 1987). To confirm that the specificity would be seen in vivo, this packaging genome is used to make infectious vector particles, and infection of and integration into estrogen-sensitive and estrogen-nonsensitive cells compared in culture.

Through use of this technique, incoming viral vectors may be directed to integrate into preselected sites on the target cell's genome, dictated by the genome-binding properties of site-specific DNA-binding protein segments spliced into the integrase genome. It will be understood by those skilled in the art that the integration site must, in fact, be receptive to the fingers of the modified integrase. For example, most cells are sensitive to glucocorticoids and hence their chromatin has sites for glucocorticoid receptors. Thus, for most cells, a modified integrase having a glucocorticoid receptor finger would be suitable to integrate the proviral vector construct at those glucocorticoid receptor-binding sites.

### Production of Recombinant Retroviral Vectors in Transgenic Animals

Two problems previously described with helper line generation of retroviral vectors are:
(a) difficulty in generating large quantities of vectors; and (b) the current need to use permanent instead of primary cells to make vectors. These problems can be overcome with producer or packaging lines that are generated in transgenic animals. These animals would carry the packaging genomes and retroviral vector genomes. Current technology does not allow the generation of packaging cell lines and desired vector-producing lines in primary cells due to their limited life span. The current technology is such that extensive characterization is necessary, which eliminates the use of primary cells because of senescence. However, individual lines of transgenic animals can be generated by the methods provided herein which produce the packaging functions, such as gag, pol or env. These lines of animals are then characterized for expression in either the whose animal or targeted tissue through the selective use of housekeeping or tissue-specific promoters to transcribe the packaging functions. The vector to be delivered is also inserted into a line of transgenic animals with a tissue-specific or housekeeping promoter. As discussed above, the vector can be driven off such a promoter substituting for the U3 region of the 5' LTR (Figure 14). This transgene could be inducible or ubiquitous in its expression. This vector, however, is not packaged. These lines of animals are then mated to the gag/pol/env animal and subsequent progeny produce packaged vector. The progeny, which are essentially identical, are characterized and offer an unlimited source of primary producing cells. Alternatively, primary cells making gag/pol and env and derived from transgenic animals can be infected or transfected in bulk with retrovirus vectors to make a primary cell producer line. Many different transgenic animals or insects could produce these vectors, such as mice, rats, chickens, swine, rabbits, cows, sheep, fish and flies. The vector and packaging genomes would be tailored for species infection specificity and tissue-specific expression through the use of tissue-specific promoters and different envelope proteins. An example of such a procedure is illustrated in Figure 15.

Although the following examples of transgenic production of primary packaging lines are described only for mice, these procedures could be extended to other species by those skilled in the art. Given the homology to MLV sequences in mice genome, the final preferred animals would not be mice.

### EXAMPLE 8

### Production of Gag-Pol Proteins Using Housekeeping Promoters for Ubiquitous Expression in Transgenic Animals

An example of a well-characterized housekeeping promoter is the HPRT promoter. HPRT is a purine salvage enzyme which expresses in all tissues. (See Patel et al., Mol. Cell Biol. 6:4-403, 1986 and Melton et al., Proc. Natl. Acad. Sci. 81:2147-2151, 1984). This promoter would be inserted in front of various gag/pol fragments (e.g., Bal I/Sca I; Aat II/Sca I; Pst I/Sca I of MoMLV; see RNA Tumor Viruses 2, 1985, Cold Spring Harbor Laboratory, 1985) that are cloned in Bluescript plasmids (Strategene, Inc.) using recombinant DNA techniques (see Maniatis et al., Molecular Cloning: A Laboratory Manual, Cold Spring Harbor, 1982). The resulting plasmids are purified (Maniatis et al., op. cit.) and the relevant genetic information isolated using Geneclean (Bio 101) or electroelution (see Hogan et al.(Eds.) Manipulating the Mouse Embryo: A Laboratory Manual, Cold Spring Harbor, 1986).

These fully characterized DNAs would be microinjected in the pronucleus of fertilized mouse ova at a concentration of 2 ug/ml. Live born mice would be screened by tail blot analyses (see Hogan et al., op. cit.). Transgenic-positive animals would be characterized for expression levels of gag-pol proteins by immunoprecipitation of radiolabeled primary cells, such as fibroblast (see Harlow et al., eds. Antibodies: A Laboratory Manual, Cold Spring Harbor, 1988). Animals would then be bred to homozygosity for establishment of animal lines that produce characterized levels of gag-pol.

### EXAMPLE 9

### Production of env Protein/Hybrid Enveloppe Proteins Using Housekeeping Promoters for Ubiquitous Expression in Transgenic Animals

This example utilizes the HPRT promoter for expression of either envelope or hybrid envelope proteins. The envelope proteins could be from any retrovirus that is capable of complementing the relevant gag-pol, in this case that of MLV. Examples are ecotropic MLV, amphotropic MLV, xenotropic MLV, polytropic MLV, or hybrid envelopes. As above, the envelope gene would be cloned behind the HPRT promoter using recombinant DNA techniques (see Maniatis et al., op. cit.). The resulting "minigene" would be isolated (see Hogan et al., op. cit.), and expression of envelope protein would be determined (Harlow et al., op. cit.). The transgenic envelope animals would be bred to homozygosity to establish a well-characterized envelope animal.

### EXAMPLE 10

### Production of gag-pol-env Animals Using Housekeeping Promoters for Ubiquitous Expression in Transgenic Animals

This would use the well-characterized gag-pol animals, as well as the animals for the establishment of a permanent gag-pol/envelope animal line. This would involve breeding to homozygosity and the establishment of a well-characterized line. These lines would then be used to establish primary mouse embryo lines that could be used for packaging vectors in tissue culture. Furthermore, animals containing the retroviral vector could be bred into this line.

### EXAMPLE 11

### Production of Tissue-Specific Expression of gag-pol-env or Hybrid Envelope in Transgenic Animals

The example given here is to direct tissue expression of the gagpol, envelope, or hybrid envelope to specific tissues, such as T-cells. This involves the use of CD2 sequences (see Lang et al., EMBO J. 7:1675-1682, 1988) that give position and copy number independence. The 1.5 kb Bam H1/Hind III fragment from the CD2 gene would be inserted in front of gag-pol, envelope, or hybrid envelope fragments using recombinant DNA techniques. These genes would be inserted into fertilized mouse ova by microinjection. Transgenic animals would be characterized as before. Expression in T-cells would be established. Animals would be bred to homozygosity to establish well-characterized lines of transgenic animals. Gag-pol animals would be mated to envelope animals to establish gag-pol-env animals expressing only in T-cells. The T-cells of these animals would then be a source for T-cells capable of packaging retroviral vectors. Again, vector animals could be bred into these gag-pol-env animals to establish T-cells expressing the vector.

This technique allows the use of other tissue-specific promoters, such as milk-specific (whey) , pancreatic (insulin or elastase), or neuronal (myelin basic protein) promoters. Through the use of promoters, such as milk-specific promoters, recombinant retroviruses may be isolated directly from the biological fluid of the progeny.

### EXAMPLE 12

### Production of Either Housekeeping or Tissue-Specific Retroviral Vectors in Transgenic Animals

The insertion of retroviruses or retroviral vectors into the germ line of transgenic animals results in little or no expression. This effect, described by Jaenisch (see Jahner et al., Nature 298:623-628, 1982), is attributed to methylation of 5' retroviral LTR sequences. This technique would overcome the methylation effect by substituting either a housekeeping or tissue-specific promoter to express the retroviral vector/retrovirus. The U3 region of the 5' LTR, which contains the enhancer elements, is replaced with regulatory sequences from housekeeping or tissue-specific promoters (see Figure 15). The 3' LTR is fully retained, as it contains sequences necessary for polyadenylation of the viral RNA and integration. As the result of unique properties of retroviral replication, the U3 region of the 5' LTR of the integrated provirus is generated by the U3 region of the 3' LTR of the infecting virus. Hence, the 3' is necessary, while the 5' U3 is dispensable. Substitution of the 5' LTR U3 sequences with promoters and insertion into the germ line of transgenic animals results in lines of animals capable of producing retroviral vector transcripts. These animals would then be mated to gag-pol-env animals to generate retroviral-producing animals (see Figure 15).

## Claims (Claims for the following Contracting State(s): AT, BE, CH, LI, DE, FR, GB, IT, LU, NL, SE)

1. A replication defective recombinant retrovirus for use in a method of therapeutic treatment, said retrovirus carrying a vector construct capable of preventing, inhibiting, stabilizing or reversing an infectious, cancerous or auto-immune disease, said vector construct when in human cells infected with said retrovirus being capable of directing the expression of an enzyme which is not normally expressed in said cells and which converts a compound with little or no cytotoxicity into a cytotoxic compound capable of inhibiting a function of a pathogenic agent necessary for pathogenicity.

2. A recombinant retrovirus according to claim 1 wherein said vector construct is capable of directing the expression of an enzyme selected from:
a. a non-mammalian protein which is not cytotoxic but which is capable of processing within a human cell a compound with little or no cytotoxicity into one which is cytotoxic;
b. a protein which is capable of phosphorylating a purine or pyrimidine-based drug which requires intracellular phosphorylation to become a cytotoxic agent; or
c. a protein which is not cytotoxic but which is capable of processing a nontoxic drug which is a purine or pyrimidine analogue into one which is cytotoxic within a human cell.

3. A recombinant retrovirus according to claim 2 wherein said vector construct is capable of directing the expression of a viral enzyme for phosphorylation of a pyrimidine-based compound.

4. A recombinant retrovirus according to claim 3 wherein said vector construct directs the expression of Herpes simplex virus thymidine kinase (HSVTK).

5. A recombinant retrovirus according to any one of claims 1 to 4 wherein expression of said enzyme by said vector construct is regulatable by the presence of an intracellular signal associated with the pathogenic state of a viral infection or a cancerous disease.

6. A recombinant retrovirus according to claim 5 wherein said vector construct has an event-specific promoter operably-linked to the vector sequence encoding said enzyme such that expression of said enzyme under the control of said promoter is regulatable by said intracellular signal.

7. A recombinant retrovirus according to claim 6 wherein said disease is a cancerous disease and wherein said event-specific promoter is preferentially active in proliferating cells.

8. A recombinant retrovirus according to any of claims 1 to 7 wherein said vector construct has a tissue-specific promoter operably-linked to the vector sequence encoding said enzyme.

9. A recombinant retrovirus according to any one of claims 1 to 8 for use in a method of diminishing or eliminating an unwanted or deleterious immune response in the human body.

10. A recombinant retrovirus according to any one of claims 1 to 8 for use in a method of inhibiting the interaction of cells with cells in the human body.

11. A recombinant retrovirus according to claim 8, wherein said disease is a cancerous disease and wherein the tissue specificity of the tissue specific promoter corresponds to the origin of the tumour.

12. A recombinant retrovirus according to claim 11, wherein said cancerous disease is a hepatocellular carcinoma and wherein said tissue-specific promoter is a liver specific promoter.

13. A recombinant retrovirus according to claim 11 or claim 12 wherein said vector construct further comprises an event-specific promoter operably-linked to the vector sequence encoding said enzyme such that the expression of said enzyme is regulatable by said event-specific promoter and said tissue specific promoter.

14. Ex vivo cells for use in a method of therapeutic treatment which are infected with a recombinant retrovirus as defined in any one of claims 1 to 13.

15. A eukaryotic cell for use in a method of therapeutic treatment which produces a replication defective recombinant retrovirus according to any one of claims 1 to 13, said cell containing a retroviral genome for said recombinant retrovirus having a packaging signal and vector sequences for expression of an enzyme as defined in any one of claims 1 to 13, but lacking the coding sequence for one or more retroviral proteins and said cell also being capable of expressing viral proteins for production of said recombinant retrovirus.

16. A product comprising:
(a) a recombinant retrovirus as defined in any one of claims 1 to 13; and
(b) a compound with little or no cytotoxicity which is capable of conversion by the enzyme encoded by the vector construct of said recombinant retrovirus to a cytotoxic compound
for use in a method of treatment of the human body.

17. A product according to claim 16 for use in treating a cancerous disease wherein expression of said enzyme is under the control of a tissue specific promoter said tissue specific promoter being active in tumour cells associated with said disease but not non-tumour cells corresponding to the origin of the tumour.

18. A product according to claim 17 wherein said tissue specific promoter is active in hepatocarcinoma cells.

19. A pharmaceutical composition comprising a replication defective recombinant retrovirus according to any one of claims 1 to 13 in combination with a pharmaceutically acceptable carrier.

20. A pharmaceutical composition comprising ex vivo cells according to claim 14 in combination with a pharmaceutically acceptable carrier.

21. A pharmaceutical composition comprising eukaryotic cells according to claim 15 in combination with a pharmaceutically acceptable carrier.

22. Use of a replication defective recombinant retrovirus according to any one of claims 1 to 13 for the manufacture of a medicament comprising cells according to claim 14 or claim 15 for use in a method of treatment of a human patient, said method comprising administering to a patient treated with said cells a compound having little or no cytotoxicity but which is converted by the enzyme expressed by the vector construct of said recombinant retrovirus to a cytotoxic compound.

23. Use of a replication defective recombinant retrovirus according to claim 22 wherein said enzyme is a viral kinase as defined in claim 3 or claim 4.

## Claims (Claims for the following Contracting State(s): ES)

1. A process for the manufacture of a medicament comprising a replication-defective recombinant retrovirus for use in a method of therapeutic treatment selected from preventing, inhibiting, stabilizing or reversing an infectious, cancerous or auto-immune disease,
said process comprising the use of a retrovirus carrying a vector construct capable of preventing, inhibiting, stabilizing or reversing an infectious, cancerous or auto-immune disease, said vector construct when in human cells infected with said retrovirus being capable of directing the expression of an enzyme which is not normally expressed in said cells and which converts a compound with little or no cytotoxicity into a cytotoxic compound capable of inhibiting a function of a pathogenic agent necessary for pathogenicity.

2. A process according to claim 1 wherein said vector construct is capable of directing the expression of an enzyme selected from:
a. a non-mammalian protein which is not cytotoxic but which is capable of processing within a human cell a compound with little or no cytotoxicity into one which is cytotoxic;
b. a protein which is capable of phosphorylating a purine or pyrimidine-based drug which requires intracellular phosphorylation to become a cytotoxic agent; or
c. a protein which is not cytotoxic but which is capable of processing a nontoxic drug which is a purine or pyrimidine analogue into one which is cytotoxic within a human cell.

3. A process according to claim 2 wherein said vector construct is capable of directing the expression of a viral enzyme for phosphorylation of a pyrimidine-based compound.

4. A process according to claim 3 wherein said vector construct directs the expression of Herpes simplex virus thymidine kinase (HSVTK).

5. A process according to any one of claims 1 to 4 wherein expression of said enzyme by said vector construct is regulatable by the presence of an intracellular signal associated with the pathogenic state of a viral infection or a cancerous disease.

6. A process according to claim 5 wherein said vector construct has an event-specific promoter operably-linked to the vector sequence encoding said enzyme such that expression of said enzyme under the control of said promoter is regulatable by said intracellular signal.

7. A process according to claim 6 wherein said disease is a cancerous disease and wherein said event-specific promoter is preferentially active in proliferating cells.

8. A process according to any of claims 1 to 7 wherein said vector construct has a tissue-specific promoter operably-linked to the vector sequence encoding said enzyme.

9. A process according to any one of claims 1 to 8 for use in a method of diminishing or eliminating an unwanted or deleterious immune response in the human body.

10. A process according to any one of claims 1 to 8 for use in a method of inhibiting the interaction of cells with cells in the human body.

11. A process according to claim 8, wherein said disease is a cancerous disease and wherein the tissue specificity of the tissue specific promoter corresponds to the origin of the tumour.

12. A process according to claim 11, wherein said cancerous disease is a hepatocellular carcinoma and wherein said tissue-specific promoter is a liver specific promoter.

13. A process according to claim 11 or claim 12 wherein said vector construct further comprises an event-specific promoter operably-linked to the vector sequence encoding said enzyme such that the expression of said enzyme is regulatable by said event-specific promoter and said tissue specific promoter.

14. A process according to any one of claims 1 to 13 for the manufacture of a medicament comprising cells, said cells being ex vivo cells infected with said retrovirus.

15. A process according to any one of claims 1 to 13 for the manufacture of a medicament comprising cells,
said cells being eukaryotic cells which produce a retrovirus as defined in any one of claims 1 to 13, said cells containing a retroviral genome for said virus having a packaging signal and vector sequences for expression of an enzyme as defined in any one of claims 1 to 13, but lacking the coding sequence for one or more retroviral proteins and said cells also being capable of expressing viral proteins for production of said retrovirus.

16. A process according to claim 14 or claim 15 wherein said enzyme is a viral kinase as defined in claim 3 or claim 4.

17. A replication-defective recombinant retrovirus for use in a method of therapeutic treatment, said retrovirus carrying a vector construct capable of preventing, inhibiting, stabilizing or reversing an infectious, cancerous or auto-immune disease, said vector construct when in human cells infected with said retrovirus being capable of directing the expression of an enzyme which is not normally expressed in said cells and which converts a compound with little or no cytotoxicity into a cytotoxic compound capable of inhibiting a function of a pathogenic agent necessary for pathogenicity.

18. A recombinant retrovirus according to claim 17 wherein said vector construct is capable of directing the expression of an enzyme selected from:
a. a non-mammalian protein which is not cytotoxic but which is capable of processing within a human cell a compound with little or no cytotoxicity into one which is cytotoxic;
b. a protein which is capable of phosphorylating a purine or pyrimidine-based drug which requires intracellular phosphorylation to become a cytotoxic agent; or
c. a protein which is not cytotoxic but which is capable of processing a nontoxic drug which is a purine or pyrimidine analogue into one which is cytotoxic within a human cell.

19. A recombinant retrovirus according to claim 18 wherein said vector construct is capable of directing the expression of a viral enzyme for phosphorylation of a pyrimidine-based compound.

20. A recombinant retrovirus according to claim 19 wherein said vector construct directs the expression of Herpes simplex virus thymidine kinase (HSVTK).

21. A recombinant retrovirus according to any one of claims 17 to 20 wherein expression of said enzyme by said vector construct is regulatable by the presence of an intracellular signal associated with the pathogenic state of a viral infection or a cancerous disease.

22. A recombinant retrovirus according to claim 21 wherein said vector construct has an event-specific promoter operably-linked to the vector sequence encoding said enzyme such that expression of said enzyme under the control of said promoter is regulatable by said intracellular signal.

23. A recombinant retrovirus according to claim 22 wherein said disease is a cancerous disease and wherein said event-specific promoter is preferentially active in proliferating cells.

24. A recombinant retrovirus according to any of claims 17 to 23 wherein said vector construct has a tissue-specific promoter operably-linked to the vector sequence encoding said enzyme.

25. A recombinant retrovirus according to any one of claims 17 to 24 for use in a method of diminishing or eliminating an unwanted or deleterious immune response in the human body.

26. A recombinant retrovirus according to any one of claims 17 to 24 for use in a method of inhibiting the interaction of cells with cells in the human body.

27. A recombinant retrovirus according to claim 24, wherein said disease is a cancerous disease and wherein the tissue specificity of the tissue specific promoter corresponds to the origin of the tumour.

28. A recombinant retrovirus according to claim 27, wherein said cancerous disease is a hepatocellular carcinoma and wherein said tissue-specific promoter is a liver specific promoter.

29. A recombinant retrovirus according to claim 27 or claim 28 wherein said vector construct further comprises an event-specific promoter operably-linked to the vector sequence encoding said enzyme such that the expression of said enzyme is regulatable by said event-specific promoter and said tissue specific promoter.

30. Ex vivo cells for use in a method of therapeutic treatment which are infected with a recombinant retrovirus as defined in any one of claims 17 to 29.

31. A eukaryotic cell for use in a method of therapeutic treatment which produces a replication defective recombinant retrovirus according to any one of claims 17 to 29, said cell containing a retroviral genome for said recombinant retrovirus having a packaging signal and vector sequences for expression of an enzyme as defined in any one of claims 17 to 29, but lacking the coding sequence for one or more retroviral proteins and said cell also being capable of expressing viral proteins for production of said recombinant retrovirus.

32. A product comprising:
(a) a recombinant retrovirus as defined in any one of claims 17 to 29; and
(b) a compound with little or no cytotoxicity which is capable of conversion by the enzyme encoded by the vector construct of said recombinant retrovirus to a cytotoxic compound
for use in a method of treatment of the human body.

33. A product according to claim 32 for use in treating a cancerous disease wherein expression of said enzyme is under the control of a tissue specific promoter said tissue specific promoter being active in tumour cells associated with said disease but not non-tumour cells corresponding to the origin of the tumour.

34. A product according to claim 33 wherein said tissue specific promoter is active in hepatocarcinoma cells.

35. A pharmaceutical composition comprising a replication defective recombinant retrovirus according to any one of claims 17 to 29 in combination with a pharmaceutically acceptable carrier.

36. A pharmaceutical composition comprising ex vivo cells according to claim 30 in combination with a pharmaceutically acceptable carrier.

37. A pharmaceutical composition comprising eukaryotic cells according to claim 31 in combination with a pharmaceutically acceptable carrier.

## Claims (Claims for the following Contracting State(s): GR)

1. Use of a replication-defective recombinant retrovirus for the manufacture of a medicament for use in a method of therapeutic treatment selected from preventing, inhibiting, stabilizing or reversing an infectious, cancerous or auto-immune disease,
said retrovirus carrying a vector construct capable of preventing, inhibiting, stabilizing or reversing an infectious, cancerous or auto-immune disease, said vector construct when in human cells infected with said retrovirus being capable of directing the expression of an enzyme which is not normally expressed in said cells and which converts a compound with little or no cytotoxicity into a cytotoxic compound capable of inhibiting a function of a pathogenic agent necessary for pathogenicity.

2. Use of a recombinant retrovirus according to claim 1 wherein said vector construct is capable of directing the expression of an enzyme selected from:
a. a non-mammalian protein which is not cytotoxic but which is capable of processing within a human cell a compound with little or no cytotoxicity into one which is cytotoxic;
b. a protein which is capable of phosphorylating a purine or pyrimidine-based drug which requires intracellular phosphorylation to become a cytotoxic agent; or
c. a protein which is not cytotoxic but which is capable of processing a nontoxic drug which is a purine or pyrimidine analogue into one which is cytotoxic within a human cell.

3. Use of a recombinant retrovirus according to claim 2 wherein said vector construct is capable of directing the expression of a viral enzyme for phosphorylation of a pyrimidine-based compound.

4. Use of a recombinant retrovirus according to claim 3 wherein said vector construct directs the expression of Herpes simplex virus thymidine kinase (HSVTK).

5. Use of a recombinant retrovirus according to any one of claims 1 to 4 wherein expression of said enzyme by said vector construct is regulatable by the presence of an intracellular signal associated with the pathogenic state of a viral infection or a cancerous disease.

6. Use of a recombinant retrovirus according to claim 5 wherein said vector construct has an event-specific promoter operably-linked to the vector sequence encoding said enzyme such that expression of said enzyme under the control of said promoter is regulatable by said intracellular signal.

7. Use of a recombinant retrovirus according to claim 6 wherein said disease is a cancerous disease and wherein said event-specific promoter is preferentially active in proliferating cells.

8. Use of a recombinant retrovirus according to any of claims 1 to 7 wherein said vector construct has a tissue-specific promoter operably-linked to the vector sequence encoding said enzyme.

9. Use of a recombinant retrovirus according to any one of claims 1 to 8 for use in a method of diminishing or eliminating an unwanted or deleterious immune response in the human body.

10. Use of a recombinant retrovirus according to any one of claims 1 to 8 for use in a method of inhibiting the interaction of cells with cells in the human body.

11. Use of a recombinant retrovirus according to claim 8, wherein said disease is a cancerous disease and wherein the tissue specificity of the tissue specific promoter corresponds to the origin of the tumour.

12. Use of a recombinant retrovirus according to claim 11, wherein said cancerous disease is a hepatocellular carcinoma and wherein said tissue-specific promoter is a liver specific promoter.

13. Use of a recombinant retrovirus according to claim 11 or claim 12 wherein said vector construct further comprises an event-specific promoter operably-linked to the vector sequence encoding said enzyme such that the expression of said enzyme is regulatable by said event-specific promoter and said tissue specific promoter.

14. Use of a replication-defective recombinant retrovirus according to any one of claims 1 to 13 for the manufacture of a medicament comprising cells, said cells being *ex vivo* cells infected with said retrovirus.

15. Use of a replication-defective recombinant retrovirus according to any one of claims 1 to 13 for the manufacture of a medicament comprising cells, said cells being eukaryotic cells which produce said virus, said cells containing a retroviral genome for said virus having a packaging signal and vector sequences for expression of an enzyme as defined in any one of claims 1 to 13, but lacking the coding sequence for one or more retroviral proteins and said cells also being capable of expressing viral proteins for production of said recombinant retrovirus.

16. Use of a replication defective recombinant retrovirus according to claim 14 or claim 15 wherein said enzyme is a viral kinase as defined in claim 3 or claim 4.

17. A replication-defective recombinant retrovirus for use in a method of therapeutic treatment, said retrovirus carrying a vector construct capable of preventing, inhibiting, stabilizing or reversing an infectious, cancerous or auto-immune disease, said vector construct when in human cells infected with said retrovirus being capable of directing the expression of an enzyme which is not normally expressed in said cells and which converts a compound with little or no cytotoxicity into a cytotoxic compound capable of inhibiting a function of a pathogenic agent necessary for pathogenicity.

18. A recombinant retrovirus according to claim 17 wherein said vector construct is capable of directing the expression of an enzyme selected from:
a. a non-mammalian protein which is not cytotoxic but which is capable of processing within a human cell a compound with little or no cytotoxicity into one which is cytotoxic;
b. a protein which is capable of phosphorylating a purine or pyrimidine-based drug which requires intracellular phosphorylation to become a cytotoxic agent; or
c. a protein which is not cytotoxic but which is capable of processing a nontoxic drug which is a purine or pyrimidine analogue into one which is cytotoxic within a human cell.

19. A recombinant retrovirus according to claim 18 wherein said vector construct is capable of directing the expression of a viral enzyme for phosphorylation of a pyrimidine-based compound.

20. A recombinant retrovirus according to claim 19 wherein said vector construct directs the expression of Herpes simplex virus thymidine kinase (HSVTK).

21. A recombinant retrovirus according to any one of claims 17 to 20 wherein expression of said enzyme by said vector construct is regulatable by the presence of an intracellular signal associated with the pathogenic state of a viral infection or a cancerous disease.

22. A recombinant retrovirus according to claim 21 wherein said vector construct has an event-specific promoter operably-linked to the vector sequence encoding said enzyme such that expression of said enzyme under the control of said promoter is regulatable by said intracellular signal.

23. A recombinant retrovirus according to claim 22 wherein said disease is a cancerous disease and wherein said event-specific promoter is preferentially active in proliferating cells.

24. A recombinant retrovirus according to any of claims 17 to 23 wherein said vector construct has a tissue-specific promoter operably-linked to the vector sequence encoding said enzyme.

25. A recombinant retrovirus according to any one of claims 17 to 24 for use in a method of diminishing or eliminating an unwanted or deleterious immune response in the human body.

26. A recombinant retrovirus according to any one of claims 17 to 24 for use in a method of inhibiting the interaction of cells with cells in the human body.

27. A recombinant retrovirus according to claim 24, wherein said disease is a cancerous disease and wherein the tissue specificity of the tissue specific promoter corresponds to the origin of the tumour.

28. A recombinant retrovirus according to claim 27, wherein said cancerous disease is a hepatocellular carcinoma and wherein said tissue-specific promoter is a liver specific promoter.

29. A recombinant retrovirus according to claim 27 or claim 28 wherein said vector construct further comprises an event-specific promoter operably-linked to the vector sequence encoding said enzyme such that the expression of said enzyme is regulatable by said event-specific promoter and said tissue specific promoter.

30. Ex vivo cells for use in a method of therapeutic treatment which are infected with a recombinant retrovirus as defined in any one of claims 17 to 29.

31. A eukaryotic cell for use in a method of therapeutic treatment which produces a replication defective recombinant retrovirus according to any one of claims 17 to 29, said cell containing a retroviral genome for said recombinant retrovirus having a packaging signal and vector sequences for expression of an enzyme as defined in any one of claims 17 to 29, but lacking the coding sequence for one or more retroviral proteins and said cell also being capable of expressing viral proteins for production of said recombinant retrovirus.

32. A product comprising:
(a) a recombinant retrovirus as defined in any one of claims 17 to 29; and
(b) a compound with little or no cytotoxicity which is capable of conversion by the enzyme encoded by the vector construct of said recombinant retrovirus to a cytotoxic compound
for use in a method of treatment of the human body.

33. A product according to claim 32 for use in treating a cancerous disease wherein expression of said enzyme is under the control of a tissue specific promoter said tissue specific promoter being active in tumour cells associated with said disease but not non-tumour cells corresponding to the origin of the tumour.

34. A product according to claim 33 wherein said tissue specific promoter is active in hepatocarcinoma cells.

35. A pharmaceutical composition comprising a replication defective recombinant retrovirus according to any one of claims 17 to 29 in combination with a pharmaceutically acceptable carrier.

36. A pharmaceutical composition comprising ex vivo cells according to claim 30 in combination with a pharmaceutically acceptable carrier.

37. A pharmaceutical composition comprising eukaryotic cells according to claim 31 in combination with a pharmaceutically acceptable carrier.

## Patentansprüche (Patentansprüche für folgende(n) Vertragsstaat(en): AT, BE, CH, LI, DE, FR, GB, IT, LU, NL, SE)

1. Replikationsdefektes rekombinantes Retrovirus zur Verwendung in einem Verfahren der therapeutischen Behandlung, wobei das Retrovirus ein Vektorkonstrukt trägt, das in der Lage ist, eine infektiöse Erkrankung, einer kanzeröse Erkrankung oder eine Autoimmunerkrankung zu inhibieren, zu stabilisieren oder umzukehren, oder diesen Erkrankungen vorzubeugen, wobei das Vektorkonstrukt, wenn es in menschlichen Zellen, die mit dem Retrovirus infiziert sind, ist, in der Lage ist, die Expression eines Enzyms zu steuern, das in diesen Zellen normalerweise nicht exprimiert wird, und das eine Verbindung mit geringer oder keiner Cytotoxizität in eine cytotoxische Verbindung umwandelt, die in der Lage ist, eine Funktion eines pathogenen Aqens, die für die Pathogenität notwendig ist, zu inhibieren.

2. Rekombinantes Retrovirus nach Anspruch 1, dadurch **gekennzeichnet**, daß das Vektorkonstrukt in der Lage ist, die Expression eines Enzyms, ausgewählt aus:
a. einem Nicht-Säuqetierprotein, das nicht cytotoxisch ist, das jedoch in der Lage ist, in einer menschlichen Zelle eine Verbindung mit geringer oder keiner Cytotoxizität in eine Verbindung umzuwandeln, die cytotoxisch ist;
b. einem Protein, das in der Lage ist, ein Purin- oder Pyrimidin-basierendes Arzneimittel, das eine intrazelluläre Phosphorylierung benötigt, um ein cytotoxisches Mittel zu werden, zu phosphorylieren; oder
c. einem Protein, das nicht cytotoxisch ist, das jedoch in der Lage ist, ein nichttoxisches Arzneimittel, bei dem es sich um ein Purin- oder Pyrimidinanalog handelt, in ein Arzneimittel, das in einer menschlichen Zelle cytotoxisch ist, umzuwandeln,
zu steuern.

3. Rekombinantes Retrovirus nach Anspruch 2, dadurch **gekennzeichnet**, daß das Vektorkonstrukt in der Lage ist, die Expression eines viralen Enzyms zur Phosphorylierung einer Pyrimidin-basierenden Verbindung zu steuern.

4. Rekombinantes Retrovirus nach Anspruch 3, dadurch **gekennzeichnet**, daß das Vektorkonstrukt die Expression der Herpes-simplex-Virus-Thymidinkinase (HSVTK) steuert.

5. Rekombinantes Retrovirus nach einem der Ansprüche 1 bis 4, dadurch **gekennzeichnet**, daß die Expression des Enzyms durch das Vektorkonstrukt durch das Vorhandensein eines intrazellulären Signals, das mit dem pathogenen Zustand einer viralen Infektion oder einer kanzerösen Erkrankung assoziiert ist, regulierbar ist.

6. Rekombinantes Retrovirus nach Anspruch 5, dadurch **gekennzeichnet**, daß das Vektorkonstrukt einen ereignisspezifischen Promotor besitzt, der funktionell mit der Vektorsequenz verbunden ist, die für das Enzym codiert, so daß die Expression des Enzyms unter der Kontrolle des Promotors durch das intrazelluläre Signal regulierbar ist.

7. Rekombinantes Retrovirus nach Anspruch 6, dadurch **gekennzeichnet**, daß die Erkrankung eine kanzeröse Erkrankung ist, und der ereignisspezifische Promotor vorzugsweise in proliferierenden Zellen aktiv ist.

8. Rekombinantes Retrovirus nach einem der Ansprüche 1 bis 7, dadurch **gekennzeichnet**, daß das Vektorkonstrukt einen gewebespezifischen Promotor besitzt, der funktionell mit der Vektorsequenz, die für das Enzym codiert, verbunden ist.

9. Rekombinantes Retrovirus nach einem der Ansprüche 1 bis 8 zur Verwendung in einem Verfahren zur Verringerung oder Eliminierung einer unerwünschten oder schädlichen Immunantwort im menschlichen Körper.

10. Rekombinantes Retrovirus nach einem der Ansprüche 1 bis 8 zur Verwendung in einem Verfahren zur Inhibierung der Zell-Zell-Interaktion im menschlichen Körper.

11. Rekombinantes Retrovirus nach Anspruch 8, dadurch **gekennzeichnet**, daß die Erkrankung eine kanzeröse Erkrankung ist und die Gewebespezifität des gewebespezifischen Promotors dem der Herkunft des Tumors entspricht.

12. Rekombinantes Retrovirus nach Anspruch 11, dadurch **gekennzeichnet**, daß die kanzeröse Erkrankung ein hepatozelluläres Karzinom ist und der gewebespezifische Promotor ein leberspezifischer Promotor ist.

13. Rekombinantes Retrovirus nach Anspruch 11 oder 12, dadurch **gekennzeichnet**, daß das Vektorkonstrukt weiterhin einen ereignisspezifischen Promotor enthält, der funktionell mit der VekLorsequenz verbunden ist, die das Enzym codiert, so daß die Expression des Enzyms durch den ereignisspezifischen Promotor und den gewebespezifischen Promotor regulierbar ist.

14. Ex-vivo-Zellen zur Verwendung in einem Verfahren der therapeutischen Behandlung, die mit einem rekombinanten Retrovirus, das wie in einem Ansprüche 1 bis 13 definiert ist, infiziert sind.

15. Eukaryotische Zelle zur Verwendung in einem Verfahren der therapeutischen Behandlung, die ein replikationsdefektes rekombinantes Retrovirus nach einem der Ansprüche 1 bis 13 produziert, wobei die Zelle ein retrovirales Genom für das rekombinante Retrovirus enthält, das ein Pakkungssignal und Vektorsequenzen zur Expression eines wie einem der Ansprüche 1 bis 13 definierten Enzyms besitzt, dem jedoch die codierende Sequenz für ein oder mehrere retrovirale Proteine fehlt, und wobei die Zelle auch in der Lage ist, virale Proteine zur Produktion des genannten rekombinanten Retrovirus zu exprimieren.

16. Produkt, umfassend:
(a) ein rekombinantes Retrovirus, das wie in einem der Ansprüche 1 bis 13 definiert ist; und
(b) eine Verbindung mit geringer oder keiner Cytotoxizität, die in der Lage ist, durch das Enzym, das von dem Vektorkonstrukt des rekombinanten Retrovirus codiert wird, in eine cytotoxische Verbindung umgewandelt zu werden, zur Verwendung in einem Verfahren zur Behandlung des menschlichen Körpers.

17. Produkt nach Anspruch 16 zur Verwendung bei der Behandlung einer kanzerösen Erkrankung, wobei die Expression des Enzyms unter der Kontrolle eines gewebespezifischen Promotors ist, wobei der gewebespezifische Promotor in Tumorzellen, die mit der Erkrankung assoziiert sind, nicht jedoch in Nicht-Tumorzellen, die der Herkunft des Tumors entsprechen, aktiv ist.

18. Produkt nach Anspruch 17, dadurch **gekennzeichnet**, daß der gewebespezifische Promotor in Hepatokarzinomzellen aktiv ist.

19. Pharmazeutische Zusammensetzung, enthaltend ein replikationsdefektes rekombinantes Retrovirus nach einem der Ansprüche 1 bis 13 in Kombination mit einem pharmazeutisch annehmbaren Träger.

20. Pharmazeutische Zusammensetzung, die ex-vivo-Zellen nach Anspruch 14 in Kombination mit einem pharmazeutisch annehmbaren Träger enthält.

21. Pharmazeutische Zusammensetzung, die eukaryotische Zellen nach Anspruch 15 in Kombination mit einem pharmazeutisch annehmbaren Träger enthält.

22. Verwendung eines replikationsdefekten rekombinanten Retrovirus nach einem der Ansprüche 1 bis 13 zur Herstellung eines Medikamentes, das Zellen nach Anspruch 14 oder Anspruch 15 enthält, zur Verwendung in einem Verfahren zur Behandlung eines Menschen, wobei das Verfahren umfaßt, daß einem Patienten, der mit den genannten Zellen behandelt wurde, eine Verbindung verabreicht wird, die eine geringe oder keine Cytotoxizität besitzt, die jedoch durch das Enzym, das von dem Vektorkonstrukt des rekombinanten Retrovirus exprimiert wird, in eine cytotoxische Verbindung umgewandelt wird.

23. Verwendung eines replikationsdefekten rekombinanten Retrovirus nach Anspruch 22, dadurch **gekennzeichnet**, daß das Enzym eine wie in Anspruch 3 oder 4 definierte virale Kinase ist.

## Patentansprüche (Patentansprüche für folgende(n) Vertragsstaat(en): ES)

1. Verfahren zur Herstellung eines Medikaments, enthaltend ein replikationsdefektes rekombinantes Retrovirus zur Verwendung in einem Verfahren zur therapeutischen Behandlung, ausgewählt aus Inhibierung, Stabilisierung oder Umkehrung einer infektiösen, kanzerösen oder Autoimmunerkrankung, und Vorbeugung vor derartigen Erkrankungen, wobei das Verfahren die Verwendung eines Retrovirus umfaßt, das ein Vektorkonstrukt trägt, das in der Lage ist, eine infektiöse, kanzeröse oder Autoimmunerkrankung zu inhibieren, zu stabilisieren oder umzukehren, wobei das Vektorkonstrukt, wenn es in menschlichen Zellen, die mit dem Retrovirus infiziert sind, ist, in der Lage ist, die Expression eines Enzyms zu steuern, das nicht normalerweise in den Zellen exprimiert wird, und das eine Verbindung mit geringer oder keiner Cytotoxizität in eine cytotoxische Verbindung umwandelt, die in der Lage ist, eine Funktion eines pathogenen Agens zu inhibieren, die für die Pathogenität notwendig ist.

2. Verfahren nach Anspruch 1, dadurch **gekennzeichnet**, daß das Vektorkonstrukt in der Lage ist, die Expression eines Enzyms, ausgewählt aus:
a. einem Nicht-Säugetierprotein, das nicht cytotoxisch ist, das jedoch in der Lage ist, in einer menschlichen Zelle eine Verbindung mit geringer oder keiner Cytotoxizität in eine Verbindung umzuwandeln, die cytotoxisch ist;
b. einem Protein, das in der Lage ist, ein Purin- oder Pyrimidin-basierendes Arzneimittel, das eine intrazelluläre Phosphorylierung benötigt, um ein cytotoxisches Mittel zu werden, zu phosphorylieren; oder
c. einem Protein, das nicht cytotoxisch ist, das jedoch in der Lage ist, ein nichttoxisches Arzneimittel, bei dem es sich um ein Purin- oder Pyrimidinanalog handelt, in ein Arzneimittel, das in einer menschlichen Zelle cytotoxisch ist, umzuwandeln,
zu steuern.

3. Verfahren nach Anspruch 2, dadurch **gekennzeichnet**, daß das Vektorkonstrukt in der Lage ist, die Expression eines viralen Enzyms zur Phosphorylierung einer Pyrimidin-basierenden Verbindung zu steuern.

4. Verfahren nach Anspruch 3, dadurch **gekennzeichnet** , daß das Vektorkonstrukt die Expression der Herpes-simplex-Virus-Thymidinkinase (HSVTK) steuert.

5. Verfahren nach einem der Ansprüche 1 bis 4, dadurch **gekennzeichnet**, daß die Expression des Enzyms durch das Vektorkonstrukt durch das Vorhandensein eines intrazellulären Signals, das mit dem pathogenen Zustand einer viralen Infektion oder einer kanzerösen Erkrankung assoziiert ist, reguliert werden kann.

6. Verfahren nach Anspruch 5, dadurch **gekennzeichnet**, daß das Vektorkonstrukt einen ereignisspezifischen Promotor besitzt, der funktionell mit der Vektorsequenz verbunden ist, die für das Enzym codiert, so daß die Expression des Enzyms unter der Kontrolle des Promotors durch das intrazelluläre Signal regulierbar ist.

7. Verfahren nach Anspruch 6, dadurch **gekennzeichnet**, daß die Erkrankung eine kanzeröse Erkrankung ist, und der ereignisspezifische Promotor vorzugsweise in proliferierenden Zellen aktiv ist.

8. Verfahren nach einem der Ansprüche 1 bis 7, dadurch **gekennzeichnet**, daß das Vektorkonstrukt einen gewebespezifischen Promotor besitzt, der funktionell mit der Vektorsequenz, die für das Enzym codiert, verbunden ist.

9. Verfahren nach einem der Ansprüche 1 bis 8 zur Verwendung in einem Verfahren zur Verringerung oder Eliminierung einer unerwünschten oder schädlichen Immunantwort im menschlichen Körper.

10. Verfahren nach einem der Ansprüche 1 bis 8 zur Verwendung in einem Verfahren zur Inhibierung der Zell-Zell-Interaktion im menschlichen Körper.

11. Verfahren nach Anspruch 8, dadurch **gekennzeichnet**, daß die Erkrankung eine kanzeröse Erkrankung ist und die Gewebespezifität des gewebepezifischen Promotors der Herkunft des Tumors entspricht.

12. Verfahren nach Anspruch 11, dadurch **gekennzeichnet**, daß die kanzeröse Erkrankung ein hepatozelluläres Karzinom ist und der gewebespezifische Promotor ein leberspezifischer Promotor ist.

13. Verfahren nach Anspruch 11 oder 12, dadurch **gekennzeichnet**, daß das Vektorkonstrukt weiterhin einen ereignisspezifischen Promotor enthält, der funktionell mit der Vektorsequenz verbunden ist, die das Enzym codiert, so daß die Expression des Enzyms durch den ereignisspezifischen Promotor und den gewebespezifischen Promotor regulierbar ist.

14. Verfahren nach einem der Ansprüche 1 bis 13 zur Herstellung eines Medikaments, das Zellen enthält, wobei die Zellen ex-vivo-Zellen sind, die mit dem Retrovirus infiziert sind.

15. Verfahren nach einem der Ansprüche 1 bis 13 zur Herstellung eines Medikaments, das Zellen enthält, wobei die Zellen eukaryotische Zellen sind, die ein Retrovirus, wie in einem der Ansprüche 1 bis 13 definiert, produzieren, wobei die Zellen ein retrovirales Genom für das Virus enthalten, das ein Verpackungssignal und Vektorsequenzen zur Expression eines wie in einem der Ansprüche 1 bis 13 definierten Enzyms enthält, dem jedoch die codierende Sequenz für ein oder mehrere retrovirale Proteine fehlt, und wobei die Zellen auch in der Lage sind, virale Proteine zur Produktion der Retroviren zu exprimieren.

16. Verfahren nach Anspruch 14 oder Anspruch 15, dadurch **gekennzeichnet**, daß das Enzym eine wie in Anspruch 3 oder Anspruch 4 definierte virale Kinase ist.

17. Replikationsdefektes rekombinantes Retrovirus zur Verwendung in einem Verfahren zur therapeutischen Behandlung, wobei das Retrovirus ein Vektorkonstrukt trägt, das in der Lage ist, eine infektiöse, kanzeröse oder Autoimmunerkrankung zu inhibieren, zu stabilisieren oder umzukehren oder einer solchen Krankheit vorzubeugen, wobei das Vektorkonstrukt, wenn es in menschlichen Zellen, die mit dem Retrovirus infiziert sind, vorhanden ist, in der Lage ist, die Expression eines Enzyms zu steuern, das nicht normalerweise in den Zellen exprimiert wird und das eine Verbindung mit geringer oder keiner Cytotoxizität in eine cytotoxische Verbindung umwandelt, die in der Lage ist, eine Funktion eines pathogenen Agens, das für die Pathogenität notwendig ist, zu inhibieren.

18. Rekombinantes Retrovirus nach Anspruch 17, dadurch **gekennzeichnet**, daß das Vektorkonstrukt in der Lage ist, die Expression eines Enzyms, ausgewählt aus:
a. einem Nicht-Säugetierprotein, das nicht cytotoxisch ist, das jedoch in der Lage ist, in einer menschlichen Zelle eine Verbindung mit geringer oder keiner Cytotoxizität in eine Verbindung umzuwandeln, die cytotoxisch ist;
b. einem Protein, das in der Lage ist, ein Purin- oder Pyrimidin-basierendes Arzneimittel, das eine intrazelluläre Phosphorylierung benötigt, um ein cytotoxisches Mittel zu werden, zu phosphorylieren; oder
c. einem Protein, das nicht cytotoxisch ist, das jedoch in der Lage ist, ein nichttoxisches Arzneimittel, bei dem es sich um ein Purin- oder Pyrimidinanalog handelt, in ein Arzneimittel, das in einer menschlichen Zelle cytotoxisch ist, umzuwandeln,
zu steuern.

19. Rekombinantes Retrovirus nach Anspruch 18, dadurch **gekennzeichnet**, daß das Vektorkonstrukt in der Lage ist, die Expression eines viralen Enzyms zur Phosphorylierung einer Pyrimidin-basierenden Verbindung zu steuern.

20. Rekombinantes Retrovirus nach Anspruch 19, dadurch **gekennzeichnet**, daß das Vektorkonstrukt die Expression der Herpes-simplex-Virus-Thymidinkinase (HSVTK) steuert.

21. Rekombinantes Retrovirus nach einem der Ansprüche 17 bis 20, dadurch **gekennzeichnet**, daß die Expression des Enzyms durch das Vektorkonstrukt durch das Vorhandensein eines intrazellulären Signals, das mit dem pathogenen Zustand einer viralen Infektion oder einer kanzerösen Erkrankung assoziiert ist, regulierbar ist.

22. Rekombinantes Retrovirus nach Anspruch 21, dadurch **gekennzeichnet**, daß das Vektorkonstrukt einen ereignisspezifischen Promotor besitzt, der funktionell mit der Vektorsequenz verbunden ist, die für das Enzym codiert, so daß die Expression des Enzyms unter der Kontrolle des Promotors durch das intrazelluläre Signal regulierbar ist.

23. Rekombinantes Retrovirus nach Anspruch 22, dadurch **gekennzeichnet**, daß die Erkrankung eine kanzeröse Erkrankung ist, und der ereignisspezifische Promotor vorzugsweise in proliferierenden Zellen aktiv ist.

24. Rekombinantes Retrovirus nach einem der Ansprüche 17 bis 23, dadurch **gekennzeichnet**, daß das Vektorkonstrukt einen gewebespezifischen Promotor besitzt, der funktionell mit der Vektorsequenz, die für das Enzym codiert, verbunden ist.

25. Rekombinantes Retrovirus nach einem der Ansprüche 17 bis 24 zur Verwendung in einem Verfahren zur Verringerung oder Eliminierung einer unerwünschten oder schädlichen Immunantwort im menschlichen Körper.

26. Rekombinantes Retrovirus nach einem der Ansprüche 17 bis 24 zur Verwendung in einem Verfahren zur Inhibierung der Zell-Zell-Interaktion im menschlichen Körper.

27. Rekombinantes Retrovirus nach Anspruch 24, dadurch **gekennzeichnet**, daß die Erkrankung eine kanzeröse Erkrankung ist und die Gewebespezifität des gewebespezifischen Promotors dem der Herkunft des Tumors entspricht.

28. Rekombinantes Retrovirus nach Anspruch 27, dadurch **gekennzeichnet**, daß die kanzeröse Erkrankung ein hepatozelluläres Karzinom ist und der gewebespezifische Promotor ein leberspezifischer Promotor ist.

29. Rekombinantes Retrovirus nach Anspruch 27 oder 28, dadurch **gekennzeichnet**, daß das Vektorkonstrukt weiterhin einen ereignisspezifischen Promotor enthält, der funktionell mit der Vektorsequenz verbunden ist, die das Enzym codiert, so daß die Expression des Enzyms durch den ereignisspezifischen Promotor und den gewebespezifischen Promotor regulierbar ist.

30. Ex-vivo-Zellen zur Verwendung in einem Verfahren der therapeutischen Behandlung, die mit einem rekombinanten Retrovirus, das wie in einem Ansprüche 17 bis 29 definiert ist, infiziert sind.

31. Eukaryotische Zelle zur Verwendung in einem Verfahren der therapeutischen Behandlung, die ein replikationsdefektes rekombinantes Retrovirus nach einem der Ansprüche 17 bis 29 produziert, wobei die Zelle ein retrovirales Genom für das rekombinante Retrovirus enthält, das ein Verpackungssignal und Vektorsequenzen zur Expression eines wie einem der Ansprüche 17 bis 29 definierten Enzyms besitzt, dem jedoch die codierende Sequenz für ein oder mehrere retrovirale Proteine fehlt, und wobei die Zelle auch in der Lage ist, virale Proteine zur Produktion des genannten rekombinanten Retrovirus zu exprimieren.

32. Produkt, umfassend:
(a) ein rekombinantes Retrovirus, das wie in einem der Ansprüche 17 bis 29 definiert ist; und
(b) eine Verbindung mit geringer oder keiner Cytotoxizität, die durch das Enzym, das von dem Vektorkonstrukt des rekombinanten Retrovirus codiert wird, in eine cytotoxische Verbindung umgewandelt werden kann,
zur Verwendung in einem Verfahren zur Behandlung des menschlichen Korpers.

33. Produkt nach Anspruch 32 zur Verwendung bei der Behandlung einer kanzerösen Erkrankung, wobei die Expression des Enzyms unter der Kontrolle eines gewebespezifischen Promotors ist, wobei der gewebespezifische Promotor in Tumorzellen, die mit der Erkrankung assoziiert sind, nicht jedoch in Nicht-Tumorzellen, die der Herkunft des Tumors entsprechen, aktiv ist.

34. Produkt nach Anspruch 33, dadurch **gekennzeichnet** , daß der gewebespezifische Promotor in Hepatokarzinomzellen aktiv ist.

35. Pharmazeutische Zusammensetzung, enthaltend ein replikationsdefektes rekombinantes Retrovirus nach einem der Ansprüche 17 bis 29 in Kombination mit einem pharmazeutisch annehmbaren Träger.

36. Pharmazeutische Zusammensetzung, die ex-vivo-Zellen nach Anspruch 30 in Kombination mit einem pharmazeutisch annehmbaren Träger enthält.

37. Pharmazeutische Zusammensetzung, die eukaryotische Zellen nach Anspruch 31 in Kombination mit einem pharmazeutisch annehmbaren Träger enthält.

## Patentansprüche (Patentansprüche für folgende(n) Vertragsstaat(en): GR)

1. Verwendung eines replikationsdefekten rekombinanten retrovirus zur Herstellung eines Medikaments zur Verwertung in einem Verfahren der therapeutischen Behandlung, ausgewählt aus Vorbeugung vor infektiösen, kanzerösen oder Autoimmunerkrankungen, Inhibierung, Stabilisierung oder Umkehrung einer infektiösen, kanzerösen oder Autoimmunerkrankung, wobei das Retrovirus ein Vektorkonstrukt trägt, das in der Lage ist, eine infektiöse, kanzeröse oder Autoimmunerkrankung zu inhibieren, zu stabilisieren oder umzukehren oder den genannten Erkrankungen vorzubeugen, wobei das Vektorkonstrukt, wenn es in menschlichen Zellen, die mit dem Retrovirus infiziert sind, ist, in der Lage ist, die Expression eines Enzyms zu steuern, das nicht normalerweise in den Zellen exprimiert wird, und das eine Verbindung mit geringer oder keiner Cytotoxizität in eine cytotoxische Verbindung umwandelt, die in der Lage ist, eine Funktion eines pathogenen Agens zu inhibieren, die für die Pathogenität notwendig ist.

2. Verwendung eines rekombinanten Retrovirus nach Anspruch 1, dadurch **gekennzeichnet**, daß das Vektorkonstrukt in der Lage ist, die Expression eines Enzyms, ausgewählt aus:
B. einem Nicht-Säugetierprotein, das nicht cytotoxish ist, das jedoch in der Lage ist, in einer menschlichen Zelle eine Verbindung mit geringer oder keiner Cytotoxizität in eine Verbindung umzuwandeln, die cytotoxisch ist;
b. einem Protein, das in der Lage ist, ein Purin- oder Pyrimidin-basierendes Arzneimittel, das eine intrazelluläre Phosphorylierung benötigt, um ein cytotoxisches Mittel zu werden, zu phosphorylieren; oder
c. einem Protein, das nicht cytotoxisch ist, das jedoch in der Lage ist, ein nichttoxisches Arzneimittel, bei dem es sich um ein Purin- oder Pyrimidinanalog handelt, in ein Arzneimittel, das in einer menschlichen Zelle cytotoxisch ist, umzuwandeln,
zu steuern.

3. Verwendung eines rekombinanten Retrovirus nach Anspruch 2, dadurch **gekennzeichnet**, daß das Vektorkonstrukt in der Lage ist, die Expression eines viralen Enzyms zur Phosphorylierung einer Pyrimidin-basierenden Verbindung zu steuern.

4. Verwendung eines rekombinanten Retrovirus nach Anspruch 3, dadurch **gekennzeichnet**, daß das Vektorkonstrukt die Expression der Herpes-simplex-Virus-Thymidinkinase (HSVTK) steuert.

5. Verwendung eines rekombinanten Retrovirus nach einem der Ansprüche 1 bis 4, dadurch **gekennzeichnet**, daß die Expression des Enzyms durch das Vektorkonstrukt durch das Vorhandensein eines intrazellulären Signals, das mit dem pathogenen Zustand einer viralen Infektion oder einer kanzerösen Erkrankung assoziiert ist, reguliert werden kann.

6. Verwendung eines rekombinanten Retrovirus nach Anspruch 5, dadurch **gekennzeichnet**, daß das Vektorkonstrukt einen ereignisspezifischen Promotor besitzt, der funktionell mit der Vektorsequenz verbunden ist, die für das Enzym codiert, so daß die Expression des Enzyms unter der Kontrolle des Promotors durch das intrazelluläre Signal regulierbar ist.

7. Verwendung eines rekombinanten Retrovirus nach Anspruch 6, dadurch **gekennzeichnet**, daß die Erkrankung eine kanzeröse Erkrankung ist, und der ereignisspezifische Promotor vorzugsweise in proliferierenden Zellen aktiv ist.

8. Verwendung eines rekombinanten Retrovirus nach einem der Ansprüche 1 bis 7, dadurch **gekennzeichnet**, daß das Vektorkonstrukt einen gewebespezifischen Promotor besitzt, der funktionell mit der Vektorsequenz, die für das Enzym codiert, verbunden ist.

9. Verwendung eines rekombinanten Retrovirus nach einem der Ansprüche 1 bis 8 zur Verwendung in einem Verfahren zur Verringerung oder Eliminierung einer unerwünschten oder schädlichen Immunantwort im menschlichen Körper.

10. Verwendung eines rekombinanten Retrovirus nach einem der Ansprüche 1 bis 8 zur Verwendung in einem Verfahren zur Inhibierung der Zell-Zell-Interaktion im menschlichen Körper.

11. Verwendung eines rekombinanten Retrovirus nach Anspruch 8, dadurch **gekennzeichnet**, daß die Erkrankung eine kanzeröse Erkrankung ist und die Gewebespezifität des gewebespezifischen Promotors dem der Herkunft des Tumors entspricht.

12. Verwendung eines rekombinanten Retrovirus nach Anspruch 11, dadurch **gekennzeichnet**, daß die kanzeröse Erkrankung ein hepatozelluläres Karzinom ist und der gewebespezifische Promotor ein leberspezifischer Promotor ist.

13. Verwendung eines rekombinanten Retrovirus nach Anspruch 11 oder 12, dadurch **gekennzeichnet**, daß das Vektorkonstrukt weiterhin einen ereignisspezifischen Promotor enthält, der funktionell mit der Vektorsequenz verbunden ist, die das Enzym codiert, so daß die Expression des Enzyms durch den ereignisspezifischen Promotor und den gewebespezifischen Promotor regulierbar ist.

14. Verwendung eines replikationsdefekten rekombinanten Retrovirus nach einem der Ansprüche 1 bis 13 zur Herstellung eines Medikaments, das Zellen enthält, wobei die Zellen ex-vivo-Zellen sind, die mit dem Retrovirus infiziert sind.

15. Verwendung eines replikationsdefekten rekombinanten Retrovirus nach einem der Ansprüche 1 bis 13 zur Herstellung eines Medikaments, das Zellen enthält, wobei die Zellen eukaryotische Zellen sind, die das Virus produzieren, wobei die Zellen ein retrovirales Genom für das Virus enthalten, das ein Verpackungssignal und Vektorsequenzen zur Expression eines wie in einem der Ansprüche 1 bis 13 definierten Enzyms enthält, dem jedoch die codierende Sequenz für ein oder mehrere retrovirale Proteine fehlt, und wobei die Zellen auch in der Lage sind, virale Proteine zur Produktion der rekombinanten Retroviren zu exprimieren.

16. Verwendung eines replikationsdefekten rekombinanten Retrovirus nach Anspruch 14 oder Anspruch 15, dadurch **gekennzeichnet**, daß das Enzym eine wie in Anspruch 3 oder Anspruch 4 definierte virale Kinase ist.

17. Replikationsdefektes rekombinantes Retrovirus zur Verwendung in einem Verfahren zur therapeutischen Behandlung, wobei das Retrovirus ein Vektorkonstrukt trägt, das in der Lage ist, eine infektiöse, kanzeröse oder Autoimmunerkrankung zu inhibieren, zu stabilisieren oder umzukehren oder einer solchen Krankheit vorzubeugen, wobei das Vektorkonstrukt, wenn es in menschlichen Zellen, die mit dem Retrovirus infiziert sind, vorhanden ist, in der Lage ist, die Expression eines Enzyms zu steuern, das nicht normalerweise in den Zellen exprimiert wird und das eine Verbindung mit geringer oder keiner Cytotoxizität in eine cytotoxische Verbindung umwandelt, die in der Lage ist, eine Funktion eines pathogenen Agens, die für die Pathogenität notwendig ist, zu inhibieren.

18. Rekombinantes Retrovirus nach Anspruch 17, dadurch **gekennzeichnet**, daß das Vektorkonstrukt in der Lage ist, die Expression eines Enzyms, ausgewählt aus:
a. einem Nicht-Säugetierprotein, das nicht cytotoxisch ist, das jedoch in der Lage ist, in einer menschlichen Zelle eine Verbindung mit geringer oder keiner Cytotoxizität in eine Verbindung umzuwandeln, die cytotoxisch ist;
b. einem Protein, das in der Lage ist, ein Purin- oder Pyrimidin-basierendes Arzneimittel, das eine intrazelluläre Phosphorylierung benötigt, um ein cytotoxisches Mittel zu werden, zu phosphorylieren; oder
c. einem Protein, das nicht cytotoxisch ist, das jedoch in der Lage ist, ein nichttoxisches Arzneimittel, bei dem es sich um ein Purin- oder Pyrimidinanalog handelt, in ein Arzneimittel, das in einer menschlichen Zelle cytotoxisch ist, umzuwandeln,
zu steuern.

19. Rekombinantes Retrovirus nach Anspruch 18, dadurch **gekennzeichnet**, daß das Vektorkonstrukt in der Lage ist, die Expression eines viralen Enzyms zur Phosphorylierung einer Pyrimidin-basierenden Verbindung zu steuern.

20. Rekombinantes Retrovirus nach Anspruch 19, dadurch **gekennzeichnet**, daß das Vektorkonstrukt die Expression der Herpes-simplex-Virus-Thymidinkinase (HSVTK) steuert.

21. Rekombinantes Retrovirus nach einem der Ansprüche 17 bis 20, dadurch **gekennzeichnet**, daß die Expression des Enzyms durch das Vektorkonstrukt durch das Vorhandensein eines intrazellulären Signals, das mit dem pathogenen Zustand einer viralen Infektion oder einer kanzerösen Erkrankung assoziiert ist, regulierbar ist.

22. Rekombinantes Retrovirus nach Anspruch 21, dadurch **gekennzeichnet**, daß das Vektorkonstrukt einen ereignisspezifischen Promotor besitzt, der funktionell mit der Vektorsequenz verbunden ist, die für das Enzym codiert, so daß die Expression des Enzyms unter der Kontrolle des Promotors durch das intrazelluläre Signal regulierbar ist.

23. Rekombinantes Retrovirus nach Anspruch 22, dadurch **gekennzeichnet**, daß die Erkrankung eine kanzeröse Erkrankung ist, und der ereignisspezifische Promotor vorzugsweise in proliferierenden Zellen aktiv ist.

24. Rekombinantes Retrovirus nach einem der Ansprüche 17 bis 23, dadurch **gekennzeichnet**, daß das Vektorkonstrukt einen gewebespezifischen Promotor besitzt, der funktionell mit der Vektorsequenz, die für das Enzym codiert, verbunden ist.

25. Rekombinantes Retrovirus nach einem der Ansprüche 17 bis 24 zur Verwendung in einem Verfahren zur Verringerung oder Eliminierung einer unerwünschten oder schädlichen Immunantwort im menschlichen Körper.

26. Rekombinantes Retrovirus nach einem der Ansprüche 17 bis 24 zur Verwendung in einem Verfahren zur Inhibierung der Zell-Zell-Interaktion im menschlichen Körper.

27. Rekombinantes Retrovirus nach Anspruch 24, dadurch **gekennzeichnet**, daß die Erkrankung eine kanzeröse Erkrankung ist und die Gewebespezifität des gewebespezifischen Promotors dem der Herkunft des Tumors entspricht.

28. Rekombinantes Retrovirus nach Anspruch 27, dadurch **gekennzeichnet**, daß die kanzeröse Erkrankung ein hepatozelluläres Karzinom ist und der gewebespezifische Promotor ein leberspezifischer Promotor ist.

29. Rekombinantes Retrovirus nach Anspruch 27 oder 28, dadurch **gekennzeichnet** , daß das Vektorkonstrukt weiterhin einen ereignisspezifischen Promotor enthält, der funktionell mit der Vektorsequenz verbunden ist, die das Enzym codiert, so daß die Expression des Enzyms durch den ereignisspezifischen Promotor und den gewebespezifischen Promotor regulierbar ist.

30. Ex-vivo-Zellen zur Verwendung in einem Verfahren der therapeutischen Behandlung, die mit einem rekombinanten Retrovirus, das wie in einem Ansprüche 17 bis 29 definiert ist, infiziert sind.

31. Eukaryotische Zelle zur Verwendung in einem Verfahren der therapeutischen Behandlung, die ein replikationsdefektes rekombinantes Retrovirus nach einem der Ansprüche 17 bis 29 produziert, wobei die Zelle ein retrovirales Genom für das rekombinante Retrovirus enthält, das ein Verpackungssignal und Vektorsequenzen zur Expression eines wie einem der Ansprüche 17 bis 29 definierten Enzyms besitzt, dem jedoch die codierende Sequenz für ein oder mehrere retrovirale Proteine fehlt, und wobei die Zelle auch in der Lage ist, virale Proteine zur Produktion des genannten rekombinanten Retrovirus zu exprimieren.

32. Produkt, umfassend:
(a) ein rekombinantes Retrovirus, das wie in einem der Ansprüche 17 bis 29 definiert ist; und
(b) eine Verbindung mit geringer oder keiner Cytotoxizität, die durch das Enzym, das von dem Vektorkonstrukt des rekombinanten Retrovirus codiert wird, in eine cytotoxische Verbindung umgewandelt werden kann,
zur Verwendung in einem Verfahren zur Behandlung des menschlichen Körpers.

33. Produkt nach Anspruch 32 zur Verwendung bei der Behandlung einer kanzerösen Erkrankung, wobei die Expression des Enzyms unter der Kontrolle eines gewebespezifischen Promotors ist, wobei der gewebespezifische Promotor in Tumorzellen, die mit der Erkrankung assoziiert sind, nicht jedoch in Nicht-Tumorzellen, die der Herkunft des Tumors entsprechen, aktiv ist.

34. Produkt nach Anspruch 33, dadurch **gekennzeichnet**, daß der gewebespezifische Promotor in Hepatokarzinomzellen aktiv ist.

35. Pharmazeutische Zusammensetzung, enthaltend ein replikationsdefektes rekombinantes Retrovirus nach einem der Ansprüche 17 bis 29 in Kombination mit einem pharmazeutisch annehmbaren Träger.

36. Pharmazeutische Zusammensetzung, die ex-vivo-Zellen nach Anspruch 30 in Kombination mit einem pharmazeutisch annehmbaren Träger enthält.

37. Pharmazeutische Zusammensetzung, die eukaryotische Zellen nach Anspruch 31 in Kombination mit einem pharmazeutisch annehmbaren Träger enthält.

## Revendications (Revendications pour l'(les) Etat(s) contractant(s) suivant(s): AT, BE, CH, LI, DE, FR, GB, IT, LU, NL, SE)

1. Rétrovirus recombinant incapable de se répliquer utilisable dans une méthode de traitement thérapeutique, ledit rétrovirus portant une construction de vecteur capable d'empêcher, d'inhiber, de stabiliser une maladie infectieuse, cancéreuse ou auto-immune, ou d'en inverser le cours, ladite construction de vecteur, lorsqu'elle est dans des cellules humaines infectées par ledit rétrovirus, étant capable de commander l'expression d'une enzyme qui n'est normalement pas exprimée dans lesdites cellules et qui convertit un composé à cytotoxicité faible ou nulle en un composé cytotoxique capable d'inhiber chez un agent pathogène une fonction nécessaire pour l'effet pathogène.

2. Rétrovirus recombinant selon la revendication 1 dans lequel ladite construction de vecteur est capable de commander l'expression d'une enzyme choisie parmi :
a. une protéine ne provenant pas d'un mammifère qui n'est pas cytotoxique mais qui est capable d'effectuer la transformation dans une cellule humaine d'un composé à cytotoxicité faible ou nulle en un composé qui est cytotoxique ;
b. une protéine qui est capable de phosphoryler un médicament à base de purine ou de pyrimidine qui nécessite une phosphorylation intracellulaire pour devenir un agent cytotoxique ; ou
c. une protéine qui n'est pas cytotoxique mais qui est capable d'effectuer la transformation d'un médicament non toxique qui est un analogue de purine ou de pyrimidine en un médicament qui est cytotoxique dans une cellule humaine.

3. Rétrovirus recombinant selon la revendication 2 dans lequel ladite construction de vecteur est capable de commander l'expression d'une enzyme virale permettant la phosphorylation d'un composé à base de pyrimidine.

4. Rétrovirus recombinant selon la revendication 3 dans lequel ladite construction de vecteur commande l'expression de la thymidine kinase du virus de l'Herpes simplex (HSVTK).

5. Rétrovirus recombinant selon l'une quelconque des revendications 1 à 4 dans lequel l'expression de ladite enzyme par ladite construction de vecteur peut être régulée par la présence d'un signal intracellulaire associé à l'état pathogène d'une infection virale ou d'une maladie cancéreuse.

6. Rétrovirus recombinant selon la revendication 5 dans lequel ladite construction de vecteur a un promoteur spécifique d'un événement lié de manière fonctionnelle à la séquence de vecteur codant pour ladite enzyme de façon que l'expression de ladite enzyme sous le contrôle dudit promoteur puisse être régulée par ledit signal intracellulaire.

7. Rétrovirus recombinant selon la revendication 6, ladite maladie étant une maladie cancéreuse, et dans lequel ledit promoteur spécifique d'un événement est prétérentiellement actif clans les cellules en prolifération.

8. Rétrovirus recombinant selon l'une quelconque des revendications 1 à 7 dans lequel ladite construction de vecteur a un promoteur spécifique d'un tissu lié de manière fonctionnelle à la séquence du vecteur codant pour ladite enzyme.

9. Rétrovirus recombinant selon l'une quelconque des revendications 1 à 8 utilisable dans une méthode destinée à diminuer ou à éliminer une réponse immunitaire non souhaitée ou nuisible dans le corps humain.

10. Rétrovirus recombinant selon l'une quelconque des revendications 1 à 8 utilisable dans une méthode destinée à inhiber l'interaction de cellules avec des cellules dans le corps humain.

11. Rétrovirus recombinant selon la revendication 8, ladite maladie étant une maladie cancéreuse, et dans lequel la spécificité tissulaire du promoteur spécifique d'un tissu correspond à l'origine de la tumeur.

12. Rétrovirus recombinant selon la revendication 11, ladite maladie cancéreuse étant un carcinome hépatocellulaire, et dans lequel ledit promoteur spécifique d'un tissu est un promoteur spécifique du foie.

13. Rétrovirus recombinant selon la revendication 11 ou la revendication 12 dans lequel ladite construction de vecteur comprend en outre un promoteur spécifique d'un événement lié de manière fonctionnelle à la séquence du vecteur codant pour ladite enzyme de façon que l'expression de ladite enzyme puisse être régulée par ledit promoteur spécifique d'un événement et ledit promoteur spécifique d'un tissu.

14. Cellules ex vivo utilisables dans une méthode de traitement thérapeutique, qui sont infectées par un rétrovirus recombinant comme défini dans l'une quelconque des revendications 1 à 13.

15. Cellule eucaryote utilisable dans une méthode de traitement thérapeutique qui produit un rétrovirus recombinant incapable de se répliquer selon l'une quelconque des revendications 1 à 13, ladite cellule contenant un génome rétroviral pour ledit rétrovirus recombinant ayant un signal d'encapsidation et des séquences de vecteur pour l'expression d'une enzyme tel que défini dans l'une quelconque des revendications 1 à 13, mais dépourvu de la séquence codant pour une ou plusieurs protéines rétrovirales et ladite cellule étant également capable d'exprimer des protéines virales pour la production dudit rétrovirus recombinant.

16. Produit comprenant :
(a) un rétrovirus recombinant tel que défini dans l'une quelconque des revendications 1 à 13 ; et
(b) un composé à cytotoxicité faible ou nulle qui peut être converti par l'enzyme codée par la construction de vecteur dudit rétrovirus recombinant en un composé cytotoxique
utilisable dans une méthode de traitement du corps humain.

17. Produit selon la revendication 16 utilisable dans le traitement d'une maladie cancéreuse dans lequel l'expression de ladite enzyme est sous le contrôle d'un promoteur spécifique d'un tissu, ledit promoteur spécifique d'un tissu étant actif dans des cellules tumorales associées à ladite maladie mais pas dans des cellules non tumorales correspondant à l'origine de la tumeur.

18. Produit selon la revendication 17 dans lequel ledit promoteur spécifique d'un tissu est actif dans des cellules d'hépatocarcinome.

19. Composition pharmaceutique comprenant un rétrovirus recombinant incapable de se répliquer selon l'une quelconque des revendications 1 à 13 en combinaison avec un véhicule pharmaceutiquement acceptable.

20. Composition pharmaceutique comprenant des cellules ex vivo selon la revendication 14 en combinaison avec un véhicule pharmaceutiquement acceptable.

21. Composition pharmaceutique comprenant des cellules eucaryotes selon la revendication 15 en combinaison avec un véhicule pharmaceutiquement acceptable.

22. Utilisation d'un rétrovirus recombinant incapable de se répliquer selon l'une quelconque des revendications 1 à 13 pour la fabrication d'un médicament comprenant des cellules selon la revendication 14 ou la revendication 15 pour utilisation dans une méthode de traitement d'un patient humain, ladite méthode comprenant l'administration à un patient traité avec lesdites cellules d'un composé ayant une cytotoxicité faible ou nulle mais qui est converti par l'enzyme exprimée par la construction de vecteur dudit rétrovirus recombinant en un composé cytotoxique.

23. Utilisation d'un rétrovirus recombinant incapable de se répliquer selon la revendication 22 dans laquelle ladite enzyme est une kinase virale telle que définie à la revendication 3 ou à la revendication 4.

## Revendications (Revendications pour l'(les) Etat(s) contractant(s) suivant(s): GR)

1. Utilisation d'un rétrovirus recombinant incapable de se répliquer pour la préparation d'un médicament utilisable dans une méthode de traitement thérapeutique choisie parmi la prévention, l'inhibition, la stabilisation ou l'inversion du cours d'une maladie infectieuse, cancéreuse ou auto-immune,
ledit rétrovirus portant une construction de vecteur capable d'empêcher, d'inhiber, de stabiliser une maladie infectieuse, cancéreuse ou auto-immune, ou d'en inverser le cours, ladite construction de vecteur, lorsqu'elle est dans des cellules humaines infectées par ledit rétrovirus, étant capable de commander l'expression d'une enzyme qui n'est normalement pas exprimée dans lesdites cellules et qui convertit un composé à cytotoxicité faible ou nulle en un composé cytotoxique capable d'inhiber chez un agent pathogène une fonction nécessaire pour l'effet pathogène.

2. Utilisation d'un rétrovirus recombinant selon la revendication 1 dans laquelle ladite construction de vecteur est capable de commander l'expression d'une enzyme choisie parmi :
a. une protéine ne provenant pas d'un mammifère qui n'est pas cytotoxique mais qui est capable d'effectuer la transformation dans une cellule humaine d'un composé à cytotoxicité faible ou nulle en un composé qui est cytotoxique ;
b. une protéine qui est capable de phosphoryler un médicament à base de purine ou de pyrimidine qui nécessite une phosphorylation intracellulaire pour devenir un agent cytotoxique ; ou
c. une protéine qui n'est pas cytotoxique mais qui est capable d'effectuer la transformation d'un médicament non toxique qui est un analogue de purine ou de pyrimidine en un médicament qui est cytotoxique dans une cellule humaine.

3. Utilisation d'un rétrovirus recombinant selon la revendication 2 dans laquelle ladite construction de vecteur est capable de commander l'expression d'une enzyme virale permettant la phosphorylation d'un composé à base de pyrimidine.

4. Utilisation d'un rétrovirus recombinant selon la revendication 3 dans laquelle ladite construction de vecteur commande l'expression de la thymidine kinase du virus de l'Herpes simplex (HSVTK).

5. Utilisation d'un rétrovirus recombinant selon l'une quelconque des revendications 1 à 4 dans laquelle l'expression de ladite enzyme par ladite construction de vecteur peut être régulée par la présence d'un signal intracellulaire associé à l'état pathogène d'une infection virale ou d'une maladie cancéreuse.

6. Utilisation d'un rétrovirus recombinant selon la revendication 5 dans laquelle ladite construction de vecteur a un promoteur spécifique d'un événement lié de manière fonctionnelle à la séquence de vecteur codant pour ladite enzyme de façon que l'expression de ladite enzyme sous le contrôle dudit promoteur puisse être régulée par ledit signal intracellulaire.

7. Utilisation d'un rétrovirus recombinant selon la revendication 6 dans laquelle ladite maladie est une maladie cancéreuse et dans laquelle ledit promoteur spécifique d'un événement est préférentiellement actif dans des cellules en prolifération.

8. Utilisation d'un rétrovirus recombinant selon l'une quelconque des revendications 1 à 7 dans laquelle ladite construction de vecteur a un promoteur spécifique d'un tissu lié de manière fonctionnelle à la séquence du vecteur codant pour ladite enzyme.

9. Utilisation d'un rétrovirus recombinant selon l'une quelconque des revendications 1 à 8 destiné à être utilisé dans une méthode pour diminuer ou éliminer une réponse immunitaire non souhaitée ou nuisible dans le corps humain.

10. Utilisation d'un rétrovirus recombinant selon l'une quelconque des revendications 1 à 8 destiné à être utilisé dans une méthode pour inhiber l'interaction de cellules avec des cellules dans le corps humain.

11. Utilisation d'un rétrovirus recombinant selon la revendication 8, dans laquelle ladite maladie est une maladie cancéreuse et dans laquelle la spécificité tissulaire du promoteur spécifique d'un tissu correspond à l'origine de la tumeur.

12. Utilisation d'un rétrovirus recombinant selon la revendication 11, dans laquelle ladite maladie cancéreuse est un carcinome hépatocellulaire et dans laquelle ledit promoteur spécifique d'un tissu est un promoteur spécifique du foie.

13. Utilisation d'un rétrovirus recombinant selon la revendication 11 ou la revendication 12 dans laquelle ladite construction de vecteur comprend en outre un promoteur spécifique d'un événement lié de manière fonctionnelle à la séquence du vecteur codant pour ladite enzyme de façon que l'expression de ladite enzyme puisse être régulée par ledit promoteur spécifique d'un événement et ledit promoteur spécifique d'un tissu.

14. Utilisation d'un rétrovirus recombinant incapable de se répliquer selon l'une quelconque des revendications 1 à 13 pour la fabrication d'un médicament comprenant des cellules, lesdites cellules étant des cellules ex vivo infectées avec ledit rétrovirus.

15. Utilisation d'un rétrovirus recombinant incapable de se répliquer selon l'une quelconque des revendications 1 à 13 pour la fabrication d'un médicament comprenant des cellules, lesdites cellules étant des cellules eucaryotes qui produisent ledit virus, lesdites cellules contenant un génome rétroviral pour ledit virus ayant un signal d'encapsidation et des séquences de vecteur pour l'expression d'une enzyme, tel que défini dans l'une quelconque des revendications 1 à 13, mais étant dépourvu de la séquence codant pour une ou plusieurs protéines rétrovirales et lesdites cellules étant également capables d'exprimer des protéines virales pour la production dudit rétrovirus recombinant.

16. Utilisation d'un rétrovirus recombinant incapable de se répliquer selon la revendication 14 ou la revendication 15 dans laquelle ladite enzyme est une kinase virale telle que définie à la revendication 3 ou à la revendication 4.

17. Rétrovirus recombinant incapable de se répliquer utilisable dans une méthode de traitement thérapeutique, ledit rétrovirus portant une construction de vecteur capable d'empêcher, d'inhiber, de stabiliser une maladie infectieuse, cancéreuse ou auto-immune, ou d'en inverser le cours, ladite construction de vecteur, lorsqu'elle est dans des cellules humaines infectées par ledit rétrovirus, étant capable de commander l'expression d'une enzyme qui n'est normalement pas exprimée dans lesdites cellules et qui convertit un composé à cytotoxicité faible ou nulle en un composé cytotoxique capable d'inhiber chez un agent pathogène une fonction nécessaire pour l'effet pathogène.

18. Rétrovirus recombinant selon la revendication 17 dans lequel ladite construction de vecteur est capable de commander l'expression d'une enzyme choisie parmi :
a. une protéine ne provenant pas d'un mammifère qui n'est pas cytotoxique mais qui est capable d'effectuer la transformation dans une cellule humaine d'un composé à cytotoxicité faible ou nulle en un composé qui est cytotoxique ;
b. une protéine qui est capable de phosphoryler un médicament à base de purine ou de pyrimidine qui nécessite une phosphorylation intracellulaire pour devenir un agent cytotoxique ; ou
c. une protéine qui n'est pas cytotoxique mais qui est capable d'effectuer la transformation d'un médicament non toxique qui est un analogue de purine ou de pyrimidine en un médicament qui est cytotoxique dans une cellule humaine.

19. Rétrovirus recombinant selon la revendication 18 dans lequel ladite construction de vecteur est capable de commander l'expression d'une enzyme virale permettant la phosphorylation d'un composé à base de pyrimidine.

20. Rétrovirus recombinant selon la revendication 19 dans lequel ladite construction de vecteur commande l'expression de la thymidine kinase du virus de l'Herpes simplex (HSVTK) .

21. Rétrovirus recombinant selon l'une quelconque des revendications 17 à 20 dans lequel l'expression de ladite enzyme par ladite construction de vecteur peut être régulée par la présence d'un signal intracellulaire associé à l'état pathogène d'un infection virale ou d'une maladie cancéreuse.

22. Rétrovirus recombinant selon la revendications 21 dans lequel ladite construction de vecteur a un promoteur spécifique d'un événement lié de manière fonctionnelle à la séquence de vecteur codant pour ladite enzyme de façon que l'expression de ladite enzyme sous le contrôle dudit promoteur puisse être régulée par ledit signal intracellulaire.

23. Utilisation d'un rétrovirus recombinant selon la revendication 22 dans laquelle ladite maladie est une maladie cancéreuse et dans laquelle ledit promoteur spécifique d'un événement est préférentiellement actif dans des cellules en prolifération.

24. Rétrovirus recombinant selon l'une quelconque des revendications 17 à 23 dans lequel ladite construction de vecteur a un promoteur spécifique d'un tissu lié de manière fonctionnelle à la séquence du vecteur codant pour ladite enzyme.

25. Rétrovirus recombinant selon l'une quelconque des revendications 17 à 24 destiné à être utilisé dans une méthode pour diminuer ou éliminer une réponse immunitaire non souhaitée ou nuisible dans le corps humain.

26. Rétrovirus recombinant selon l'une quelconque des revendications 17 à 24 destiné à être utilisé dans une méthode pour inhiber l'interaction de cellules avec des cellules dans le corps humain.

27. Rétrovirus recombinant selon la revendication 24, dans laquelle ladite maladie est une maladie cancéreuse et dans laquelle la spécificité tissulaire du promoteur spécifique d'un tissu correspond à l'origine de la tumeur.

28. Rétrovirus recombinant selon la revendication 27, dans lequel ladite maladie cancéreuse est un carcinome hépatocellulaire et dans laquelle ledit promoteur spécifique d'un tissu est un promoteur spécifique du foie.

29. Rétrovirus recombinant selon la revendication 27 ou la revendication 28 dans lequel ladite construction de vecteur comprend en outre un promoteur spécifique d'un événement lié de manière fonctionnelle à la séquence du vecteur codant pour ladite enzyme de façon que l'expression de ladite enzyme puisse être régulée par ledit promoteur spécifique d'un événement et ledit promoteur spécifique d'un tissu.

30. Cellules ex vivo utilisables dans une méthode de traitement thérapeutique qui sont infectées par un rétrovirus recombinant tel que défini selon l'une quelconque des revendications 17 à 29.

31. Cellule eucaryote utilisable dans une méthode de traitement thérapeutique qui produit un rétrovirus recombinant incapable de se répliquer selon l'une quelconque des revendications 17 à 29, ladite cellule contenant un génome rétroviral pour ledit rétrovirus recombinant ayant un signal d'encapsidation et des séquences de vecteur pour l'expression d'une enzyme selon l'une quelconque des revendications 17 à 29, mais étant dépourvus de la séquence codant pour une ou plusieurs protéines rétrovirales et lesdites cellules étant également capables d'exprimer des protéines virales pour la production dudit rétrovirus recombinant.

32. Produit comprenant :
(a) un rétrovirus recombinant tel que défini selon l'une quelconque des revendications 17 à 29 ; et
(b) un composé avec peu ou pas de cytotoxicité qui est capable d'être converti, par l'enzyme encodée par la construction de vecteur dudit rétrovirus recombinant, en un composé cytotoxique,
pour son utilisation dans une méthode de traitement du corps humain.

33. Produit selon la revendication 32 pour son utilisation dans le traitement d'une maladie cancéreuse dans laquelle l'expression dudit enzyme est sous le contrôle d'un promoteur spécifique tissulaire, ledit promoteur spécifique tissulaire étant actif dans les cellules tumorales associées à ladite maladie mais pas dans les non tumorales correspondantes à l'origine de la tumeur.

34. Produit selon la revendication 33 dans lequel ledit promoteur spécifique tissulaire est actif dans les cellules hépatocarcinomes.

35. Composition pharmaceutique comprenant un rétrovirus recombinant incapable de se répliquer selon l'une quelconque des revendications 17 à 29 en combinaison avec un véhicule pharmaceutiquement acceptable.

36. Composition pharmaceutique comprenant ex vivo des cellules selon la revendication 30 en combinaison avec un véhicule pharmaceutiquement acceptable.

37. Composition pharmaceutique comprenant des cellules eucaryotes selon la revendication 31 en combinaison avec un véhicule pharmaceutiquement acceptable.

## Revendications (Revendications pour l'(les) Etat(s) contractant(s) suivant(s): ES)

1. Procédé pour la fabrication d'un médicament comprenant un rétrovirus recombinant incapable de se répliquer utilisable dans une méthode de traitement thérapeutique choisie parmi la prévention, l'inhibition, la stabilisation ou l'inversion du cours d'une maladie infectieuse, cancéreuse ou auto-immune, ledit procédé comprenant l'utilisation d'un rétrovirus portant une construction de vecteur capable d'empêcher, d'inhiber, de stabiliser une maladie infectieuse, cancéreuse ou auto-immune, ou d'en inverser le cours, ladite construction de vecteur, lorsqu'elle est dans des cellules humaines infectées par ledit rétrovirus, étant capable de commander l'expression d'une enzyme qui n'est normalement pas exprimée dans lesdites cellules et qui convertit un composé à cytotoxicité faible ou nulle en un composé cytotoxique capable d'inhiber chez un agent pathogène une fonction nécessaire pour l'effet pathogène.

2. Procédé selon la revendication 1 dans lequel ladite construction de vecteur est capable de commander l'expression d'une enzyme choisie parmi :
a. une protéine ne provenant pas d'un mammifère qui n'est pas cytotoxique mais qui est capable d'effectuer la transformation dans d'une cellule humaine d'un composé à cytotoxicité faible ou nulle en un composé cytotoxique ;
b. une protéine qui est capable de phosphoryler un médicament à base de purine ou de pyrimidine qui nécessite une phosphorylation intracellulaire pour devenir un agent cytotoxique ; ou
c. une protéine qui n'est pas cytotoxique mais qui est capable d'effectuer la transformation d'un médicament non toxique qui est un analogue de purine ou de pyrimidine en un composé qui est cytotoxique dans une cellule humaine.

3. Procédé selon la revendication dans lequel ladite construction de vecteur est capable de commander l'expression d'une enzyme virale permettant la phosphorylation d'un composé à base de pyrimidine.

4. Procédé selon la revendication 3 dans lequel ladite construction de vecteur commande l'expression de la thymidine kinase du virus de l'Herpes simplex (HSVTK).

5. Procédé selon l'une quelconque des revendications 1 à 4 dans lequel l'expression de ladite enzyme par ladite construction de vecteur peut être régulée par la présence d'un signal intracellulaire associé à l'état pathogène d'une infection virale ou d'une maladie cancéreuse.

6. Procédé selon la revendication 5 dans lequel ladite construction de vecteur a un promoteur spécifique d'un événement lié de manière fonctionnelle à la séquence du vecteur codant pour ladite enzyme de façon que l'expression de ladite enzyme sous le contrôle dudit promoteur puisse être régulée par ledit signal intracellulaire.

7. Procédé selon la revendication 6 dans lequel ladite maladie est une maladie cancéreuse et dans lequel ledit promoteur spécifique d'un événement est préférentiellement actif dans des cellules en prolifération.

8. Procédé selon l'une quelconque des revendication 1 à 7 dans lequel ladite construction de vecteur a un promoteur spécifique d'un tissu lié de manière fonctionnelle à la séquence du vecteur codant pour ladite enzyme.

9. Procédé selon l'une quelconque des revendications 1 à 8 utilisable dans une méthode destinée à diminuer ou à éliminer une réponse immunitaire non souhaitée ou nuisible dans le corps humain.

10. Procédé selon l'une quelconque des revendications 1 à 8 utilisable dans une méthode destinée à inhiber l'interaction de cellules avec des cellules dans le corps humain.

11. Procédé selon la revendication 8, dans lequel ladite maladie est une maladie cancéreuse et dans lequel la spécificité tissulaire du promoteur spécifique d'un tissu correspond à l'origine de la tumeur.

12. Procédé selon la revendication 11, dans lequel ladite maladie cancéreuse est un carcinome hépatocellulaire et dans lequel ledit promoteur spécifique d'un tissu est un promoteur spécifique du foie.

13. Procédé selon la revendication 11 ou la revendication 12 dans lequel ladite construction de vecteur comprend en outre un promoteur spécifique d'un événement lié de manière fonctionnelle à la séquence du vecteur codant pour ladite enzyme de façon que l'expression de ladite enzyme puisse être régulée par ledit promoteur spécifique d'un événement et ledit promoteur spécifique d'un tissu.

14. Procédé selon l'une quelconque des revendications 1 à 13 pour la fabrication d'un médicament comprenant des cellules, lesdites cellules étant des cellules ex vivo infectées par ledit rétrovirus.

15. Procédé selon l'une quelconque des revendication 1 à 13 pour la fabrication d'un médicament comprenant des cellules, lesdites cellules étant des cellules eucaryotes qui produisent un rétrovirus tel que défini dans l'une quelconque des revendications 1 à 13, lesdites cellules contenant un génome rétroviral pour ledit virus ayant un signal d'encapsidation et des séquences de vecteur pour l'expression d'une enzyme tel que défini dans l'une quelconque des revendications 1 à 13, mais étant dépourvu de la séquence codant pour une ou plusieurs protéines rétrovirales et lesdites cellules étant également capables d'exprimer des protéines virales pour la production dudit rétrovirus.

16. Procédé selon la revendication 14 ou la revendication 15 dans lequel ladite enzyme est une kinase virale telle que définie dans la revendication 3 ou la revendication 4.

17. Rétrovirus recombinant incapable de se répliquer utilisable dans une méthode de traitement thérapeutique, ledit rétrovirus portant une construction de vecteur capable d'empêcher, d'inhiber, stabiliser une maladie infectieuse, cancéreuse ou auto-immune, ou d'en inverser le cours, ladite construction de vecteur, lorsqu'elle est dans des cellules humaines infectées par ledit rétrovirus, étant capable de commander l'expression d'une enzyme qui n'est normalement pas exprimée dans lesdites cellules et qui convertit un composé à toxicité faible ou nulle en un composé capable d'inhiber chez un agent pathogène une fonction nécessaire pour l'effet pathogène.

18. Rétrovirus recombinant selon la revendication 17 dans lequel ladite construction de vecteur est capable de commander l'expression d'une enzyme choisie parmi :
a. une protéine ne provenant pas d'un mammifère qui n'est pas cytotoxique mais qui est capable d'effectuer la transformation dans une cellule humaine d'un composé à cytotoxicité faible ou nulle en un composé cytotoxique ;
b. une protéine qui est capable de phosphoryler un médicament à base de purine ou de de pyrimide qui nécessite une phosphorylation intracellulaire pour devenir un agent cytotoxique ; ou
c. une protéine qui n'est pas cytotoxique mais qui est capable d'effectuer la transformation d'un médicament non toxique qui est un analogue de purine ou de pyrimidine en un composé qui est cytotoxique dans une cellule humaine.

19. Rétrovirus recombinant selon la revendication 18 dans lequel ladite construction de vecteur est capable de commander l'expression d'une enzyme virale permettant la phosphorylation d'un composé à base de pyrimidine.

20. Rétrovirus recombinant selon la revendication 19 dans lequel ladite construction de vecteur commande l'expression de la thymidine kinase du virus de l'Herpes simplex (HSVTK).

21. Rétrovirus recombinant selon l'une quelconque des revendications 17 à 20 dans lequel l'expression de ladite enzyme par ladite construction de vecteur peut être régulée par la présence d'un signal intracellulaire associé à l'état pathogène d'une infection virale ou d'une maladite cancéreuse.

22. Rétrovirus recombinant selon la revendication 21 dans lequel ladite construction de vecteur a un promoteur spécifique d'un événement lié de manière fonctionnelle à la séquence du vecteur codant pour ladite enzyme de façon que l'expression de ladite enzyme sous le contrôle dudit promoteur puisse être régulée par ledit signal intracellulaire.

23. Rétrovirus recombinant selon la revendication 22 dans lequel ladite maladie est une maladie cancéreuse et dans lequel ledit promoteur spécifique d'un événement est préférentiellement actif dans des cellules en prolifération.

24. Rétrovirus recombinant selon l'une quelconque des revendications 17 à 23 dans lequel ladite construction de vecteur a un promoteur spécifique d'un tissu lié de manière fonctionnelle à la séquence du vecteur codant pour ladite enzyme.

25. Rétrovirus recombinant selon l'une quelconque des revendications 17 à 24 utilisable dans une méthode destinée à diminuer ou à éliminer une réponse immunitaire non souhaitée ou nuisible dans le corps humain.

26. Rétrovirus recombinant selon l'une quelconque des revendications 17 à 24 utilisable dans une méthode destinée à inhiber l'interaction de cellules avec des cellules dans le corps humain.

27. Rétrovirus recombinant selon la revendication 24, dans lequel ladite maladie est une maladie cacéreuse et dans lequel la spécificité tissulaire du promoteur spécifique d'un tissu correspond à l'origine de la tumeur.

28. Rétrovirus recombinant selon la revendication 27, dans lequel ladite maladie cancéreuse est un carcinome hépatocellulaire et dans lequel ledit promoteur spécifique d'un tissu est un promoteur spécifique du foie.

29. Rétrovirus recombinant selon la revendication 27 ou la revendication 28 dans lequel ladite construction de vecteur comprend en outre un promoteur spécifique d'un événement lié de manière fonctionnelle à la séquence du vecteur codant pour ladite enzyme de façon que l'expression de ladite enzyme puisse être régulée par ledit promoteur spécifique d'un événement et ledit promoteur spécifique d'un tissu.

30. Cellules ex vivo utilisables dans une méthode de traitement thérapeutique qui sont infectées par un rétrovirus recombinant tel que défini selon l'une quelconque des revendications 17 à 29.

31. Cellule eucaryote utilisable dans une méthode de traitement thérapeutique qui produit un rétrovirus recombinant incapable de se répliquer selon l'une quelconque des revendication 17 à 29, ladite cellule contenant un génome rétroviral pour ledit rétrovirus recombinant ayant un signal d'encapsidation et des séquences de vecteur pour l'expression d'une enzyme selon l'une quelconque des revendications 17 à 29, mais étant dépourvu de la séquence codant pour une ou plusieurs protéines rétrovirales et lesdites cellules étant également capables d'exprimer des protéines virales pour la production dudit rétrovirus recombinant.

32. Produit comprenant :
(a) un rétrovirus recombinant tel que défini selon l'une quelconque des revendications 17 à 29 ; et
(b) un composé avec peu ou pas de cytotoxicité qui est capable d'être converti, par l'enzyme encodée par la construction de vecteur dudit rétrovirus recombinant, en un composé cytotoxique,
pour son utilisation dans une méthode de traitement du corps humain.

33. Produit selon la revendication 32 pour son utilisation dans le traitement d'une maladie cancéreuse dans laquelle l'expression dudit enzyme est sous le contrôle d'un promoteur spécifique tissulaire, ledit promoteur spécifique tissulaire étant actif dans les cellules tumorales associées à ladite maladie mais pas dans les cellules non tumorales correspondantes à l'origine de la tumeur.

34. Produit selon la revendication 33 dans lequel ledit promoteur spécifique tissulaire est actif dans les cellules hépatocarcinomes.

35. Composition pharmaceutique comprenant un rétrovirus recombinant incapable de se répliquer selon l'une quelconque des revendications 17 à 29 en combinaison avec un véhicule pharmaceutiquement acceptable.

36. Composition pharmaceutique comprenant ex vivo des cellules selon la revendication 30 en combinaison avec un véhicule pharmaceutiquement acceptable.

37. Composition pharmaceutique comprenant des cellules eucaryotes selon la revendication 31 en combinaison avec un véhicule pharmaceutiquement acceptable.
